# EUROPÄISCHE PATENTSCHRIFT

(11) **EP 1 149 112 B1**
(45) Veröffentlichungstag und Bekanntmachung des Hinweises auf die Patenterteilung: **02.01.2013**
(21) Anmeldenummer: 00901593.4
(22) Anmeldetag: 26.01.2000
(51) Int. Cl.: C07K 14/47, C12N 15/00

(54) **EWING TUMOR ANTIGEN, NUKLEINSÄURE UND ANTIKÖRPER**
EWING'S TUMOUR ANTIGEN, NUCLEIC ACID AND ANTIBODIES
ANTIGENE, ACIDE NUCLEIQUE ET ANTICORPS DU SARCOME D'EWING

(30) Priorität: 29.01.1999 DE 19903495
(43) Veröffentlichungstag der Anmeldung: 31.10.2001
(73) Patentinhaber: Universität Ulm, 89069 Ulm (DE)
(72) Erfinder: Schneider, Marion, Prof. Dr., 89075 Ulm (DE); Dr. Alexander Borowski, 50668 Köln (DE)
(74) Vertreter: König, Gregor Sebastian
(86) Internationale Anmeldenummer: PCT/EP2000/000568
(87) Internationale Veröffentlichungsnummer: WO 2000/044782

(56) Entgegenhaltungen:
- HUBERT RENE S ET AL: "STEAP: A prostate-specific cell-surface antigen highly expressed in human prostate tumors." PROCEEDINGS OF THE NATIONAL ACADEMY OF SCIENCES OF THE UNITED STATES, Bd. 96, Nr. 25, 7. Dezember 1999 (1999-12-07), Seiten 14523-14528, XP002151506 Dec. 7, 1999 ISSN: 0027-8424
- ARGANI PEDRAM ET AL: "Olfactory neuroblastoma is not related to the Ewing family of tumors: Absence of EWS/FLI1 gene fusion and MIC2 expression." AMERICAN JOURNAL OF SURGICAL PATHOLOGY, Bd. 22, Nr. 4, April 1998 (1998-04), Seiten 391-398, XP000961359 ISSN: 0147-5185
- LIPINSKI M ET AL: "PHENOTYPIC CHARACTERIZATION OF EWING SARCOMA CELL LINES WITH MONOCLONAL ANTIBODIES" JOURNAL OF CELLULAR BIOCHEMISTRY, Bd. 31, Nr. 4, 1986, Seiten 289-296, XP000961326 ISSN: 0730-2312
- HESS-STUMPP H. ET AL.: "A novel cytoplasmic protein (Mr 100,00) shows a special association with junctional plaques of Sertoli cells and hepatocytes" EMBL DATABASE, 1. Oktober 1997 (1997-10-01), XP002151509 HEIDELBERG, DE in der Anmeldung erwähnt
- BOROWSKI A. ET AL.: "Isolation of a antibody-defined, tumor-specific antigen from Ewing's tumor cell lines"" EMBL DATABASE, 2. März 2000 (2000-03-02), XP002151510 HEIDELBERG, DE
- STRAUSBERG R. ET AL.: "tt22f06.x1 mRNA sequence. H. sapiens. EST" EMBL DATABASE, 27. April 1999 (1999-04-27), XP002151511 HEIDELBERG, DE

## Beschreibung

Die Erfindung betrifft eine Nukleinsäure, die für ein Polypeptid kodiert, welches von Ewing Tumor-Zellen exprimiert wird und als charakteristisches Antigen des Tumors von Antikörpern oder zytolytischen Zellen erkannt werden.

Die Ewing Tumore (ET) umfassen das rundzellige, maligne Ewing Sarkom der Plattenknochen, die Gruppe der primitiven neuroektodermalen Tumore (PNET) und die Askin's Tumore, und treten vor allem häufig bei Kindern im Alter von 5-15 Jahren auf. Sie gelten nach dem Osteosarkom als die häufigsten pädiatrischen malignen Knochentumore. Dabei zeigen speziell das Ewing Sarkom und die PNET aggressives klinisches Verhalten und bilden sehr schnell Metastasen.

Die überwiegende Mehrzahl der Ewing Tumore zeichnen sich durch eine chromosomale Translokation des langen Arms des Chromosoms 11 aus, das mit einem Abschnitt des langen Arms des Chromosomen 22 fusioniert ist t(11;22)(q24;q12). Mit dieser Translokation fusionieren die Gene fli-1 des Chromosomen 11 und ews auf Chromosomen 22. In Abhängigkeit von der Lage der Chromosomenbruchstelle können verschiedene chimäre Fusionstranskripte EWS/FLI-1 entstehen, die jeweils für die definierten Tumore charakteristisch sind. Sie scheinen für die Tumorenstehung und - progression entscheidende Bedeutung zu haben. Für detaillierte Ausführungen dazu siehe auch Aurias et al., N. Engl., J.Med. 309: 496 (1991) und Delattre et al., Nature 359: 162-165 (1992). Das Fusionsprodukt beinhaltet die transkriptionsregulierenden Elemente der beiden Ausgangsprodukte, unterscheidet sich hingegen in seinen Eigenschaften von den nativen Ausgangsproteinen. Somit zeigen die ET-Zellen Expressionsmuster, die von denen der gesunden Zellen stark abweichen.

Die ET-Patienten werden herkömmlich mit einer Kombination aus Chemotherapie und Bestrahlung behandelt. Die Prognose für Patienten mit lokalisierten kleinen Tumoren der Knochen ist mit dieser Therapie gut, da nach fünf Jahren 66% der Patienten überleben. Mit einer Überlebenschance von nur 10% ist die Prognose für Patienten mit Metastasen weitaus schlechter. Dies geht auf Untersuchungen von Jürgens et al., Cancer 61: 23-32 (1988) zurück. Ein Problem der Tumortherapie speziell beim Ewing Sarcom ist das Auftreten einer sog. Multidrug Resistenz, da ein membranständiges Transportprotein MDR-1 verschiedene applizierte Chemotherapeutika energieabhängig aus der Zelle herausschleust.

Protoonkogene, Tumorsupressorgene, Adhäsionsmoleküle und Rezeptoren für Wachstumsfaktoren stellen potentielle Antigene für eine Immuntherapie der Tumoren dar. Bei Zellen der Ewing Sarkome und der PNET ist das oberflächenexprimierte Antigen MIC als Zelladhäsionsmolekül bekannt. Beide Tumoren unterscheiden sich durch die Expression zweier neuronaler Differenzierungsantigene (NSE/s-100). Einer überwiegenden Mehrzahl der ET-Zellen ist durch Transkriptionsprodukte des Fusionsgenes *ews*/*fli-1* gekennzeichnet.

Die bisherigen Versuche der Induzierung einer Imunantwort zur Behandlung der Ewing Tumoren waren wenig erfolgreich: Bertazzoli et al., Tumori. 83: 847-855 (1997) haben Trankriptionsprodukte von *ews*/*fli-1* identifiziert, die in MHC Klasse I Moleküle passen, eine CD8⁺ spezifische T-Zellantwort konnte jedoch nicht ausgelöst werden. Shamamian et al., Cancer Immunol. Immunther. 39: 73-83 (1994) konnten erst nach einer Stimulation der MHC-Klasse I Expression eine zytotoxische T-Zellreaktion hervorrufen. Die Expression der MHC-Moleküle Klasse I ist in ET-Zellinien folglich für die Induktion einer T-Zell-Antwort zu gering.

Auch das MIC Antigen erscheint für die Immuntherapie eher ungeeignet, obwohl es speziell für die Zelladhäsion verantwortlich ist und vermutlich das Wachstum der Zellen reguliert. Es wird allerdings auch auf T-Zellen exprimiert, so daß MIC Antikörper sowohl T-Zellen als auch Tumorzellen erkennen. Für eine sinnvolle und effiziente Immuntherapie ist eine hohe Spezifität der Antigene und damit auch der Antikörperbindung aber unbedingte Voraussetzung.

Durch die Immunisierung von Balb/c Mäusen mit einem Tumorisolat gemischt mit der ET-Zellinie VH-64 und anschließender Fusion einer Milzzelle und einer NS-1 Myelomzelle gelang die Generierung monoklonaler Antikörper gegen diese Zellinie des Ewing Tumors. Weitere Analysen zeigten, daß diese Antikörper Antigene der ET-Zellen erkannten. Ein Teil der Antikörper regierte auch mit fötalen Leberzellen und entdifferenzierten Makrophagen. Nähere Einzelheiten sind dazu bei Shi et al., Cancer Immunol. Immunother. 38: 208-213 (1994) nachzulesen.

Die meisten der bisher beschriebenen tumorspezifischen Antigene (TSA) oder tumorassoziierten Antigenen (TAA), die immunologisch relevant sind, sind in Karzinomen entdeckt worden. Spezifische Antigene von Sarkomen sind bislang nur für das Melanom identifiziert. Daher ist die Suche nach TSA bzw. TAA, unabhängig davon, ob es sich um Antikörper spezifische oder T-Zell identifizierte handelt, vor allem für Sarkome, zu denen auch die Familie der Ewing Tumore gehört, essentiell für eine aussagefähige Diagnostik und erfolgreiche Immuntherapie.

Der Erfindung liegt daher die Aufgabe zugrunde, ein spezifisches Polypeptid des Ewing Tumors mit antigenischem Charakter und die diese codierende Nukleinsäure zu identifizieren.

Die Lösung der Aufgabe besteht in der Isolierung der ETAA16 cDNA gemäß SEQ.IDN aus einer cDNA Expressionsbibliothek der VH-64 Zellinie der Ewing Tumoren und der anschließenden Charakterisierung dessen Transkriptionsprodukts.

Ein Weg zur verbesserten Behandlung von Ewing Tumoren liegt in der Immuntherapie. Sowohl für die Antikörper vermittelte als auch für eine T-Zell spezifische Immunantwort sind tumorspezifische Antigene (TSA) oder tumorassoziierte Antigene (TAA) unabdingbar, die entweder de *novo* oder aber mit einer zum normalen Gewebe abweichenden Dichte exprimiert werden, und so die Tumorzellen von gesunden Zellen unterscheiden. Diese Antigene müssen entweder auf der Zelloberfläche exprimiert, oder aber in der Tumorzelle oder antigenpräsentierenden Zelle (APC) prozessiert und nachfolgend durch MHC Klasse I bzw. **II** Moleküle an der Zelloberfläche präsentiert werden.

Mit Hilfe der erfindungsgemäßen ETAA16 cDNA und dem davon codierten Antigen wird eine Immuntherapie der Ewing Tumore überhaupt erst ermöglicht. Dabei ist auch eine Verwendung der Antigene zur Weiterentwicklung einer aktiven Immunisierung denkbar. Bei prädisponierten Patienten kann diese Behandlung als Präventivmaßnahme bereits vor der eigentlichen Belastung mit dem Tumor stattfinden. Auch ist es möglich, die Regression bereits etablierter Tumoren durch die Applikation des ETAA16-Antigens und damit durch eine Expansion antigenspezifischer T-Zellen zu induzieren. Diese inflammatorische Antwort des Immunsystems kann helfen, die inhibitorischen Effekte des Tumors zu überwinden.

Eine weitere Einsatzmöglichkeit der erfindungsgemäßen Antigene besteht darin, durch ihre Applikation eine Stimulation der Immunantwort gegen die Ewing Tumore zu erzeugen oder zu stärken. Diese ist erreichbar, wenn das Antigen als biologischer Immunmodulator wirksam ist.

Die Verwendungsmöglichkeiten des ETAA16-Antigens erschöpfen sich nicht in der direkten Applikation desselben, sondern erstrecken sich vor allem auf den besonders wesentlichen Bereich der Generierung antigenspezifischer, monoklonaler Antikörper. Damit eröffnet sich das gesamte Gebiet der passiven Immuntherapie. Dies ist um so wichtiger, als daß bislang keine TSA oder TAA für die Ewing Tumore bekannt sind, und so der Einsatz spezifischer monoklonaler Antikörper für eine effiziente Immuntherapie unmöglich war. Dabei sind transplantationsmedizinische Methoden wie z.B. die Säuberung von autologen Stammzellpräperaten und Knochenmark möglich. Eine Steigerung des therapeutischen Effekts, insbesondere der Komplement-vermittelten und spezifischen Zytotoxizität, wird durch eine Kopplung der Antikörper mit Wirkstoffen, Wirkstoffvorstufen, (Exo-)Toxinen oder anderen therapeutischen Substanzen erreicht. Auch können, vor allem zur Verbesserung der Penetration erkrankter Gewebe, nur Fragmente des monoklonalen Antikörpers appliziert oder auf ihrer Grundlage Einzeldomänenantikörper gentechnisch hergestellt werden.

Der gegen das ETAA16 Antigen gerichtete Antikörper kann neben der Immuntherapie auch in der Immundiagnostik eingesetzt werden. Er kann sowohl der Bestimmung der Antigenmengen und damit der Belastung mit dem Tumor, als auch dessen Lokalisierung und Identifizierung dienen. Hier sind *in vivo* Verfahren wie beispielsweise die Immunszintigraphie mit radioaktivmarkierten Antikörpern oder *in vitro* Verfahren, wie beispielsweise die indirekte Immunoperoxidasetechnik wichtig. Daneben findet dieser Antikörper auch einen Einsatz in den herkömmlichen immunologischen Arbeitsmethoden wie beispielsweise bei einem ELISA, der Nephelometrie, der Immunpräzipitation oder im Westerb Blot.

Die ETAA16 cDNA Sequenz dient ferner als Matritze zur Generierung spezifischer Oligonukleotide, die in der RT-PCR und in der *in-situ* PCR zur sensitiveren Diagnostik der Ewing Tumoren eingesetzt werden. Beide Verfahren erhöhen die Sensitivität in der Diagnostik gegenüber der reinen Nutzung der bisher bekannten Antikörper. Indem das ETAA16 Antigen durch die PCR nachgewiesen wird, wird eine Unterscheidung zwischen Ewing Tumoren und anderen Tumoren und gesunden Geweben erzielt. Diese genaue Differenzierung ist essentiell für die sich anschließende Therapie, so daß sich die Heilungschancen des Patienten erheblich verbessern.

Die vorliegende Erfindung wird durch die nachfolgenden Figuren näher erläutert:
- Fig. 1.:: Southern Blot einer humanen genomischen DNA mit ETAA 16. auf einem 0,7 % Agarosegel, nachdem 5 µg genomischer VH-64 DNA mit einem der Restriktionsenzyme Alu I, Bam HI, Eco RI, Hind III, KPN I oder Pst I behandelt, das Agarosegel geblottet und mit einer 501 bp Dra I/ Dra III Sonde aus dem Bereich des 3'- Endes von ETAA16 cDNA hybridisiert wurde.
- Fig. 2.:: FISH-Analyse der Subklone der ETAA16 cDNA, die durch interne Schnittstellen folgender Restriktionsenzyme aus der Multi Cloning Site (MCS) hergestellt wurden: Sma I (3'Δ3145), Eco RI (5'Δ471), Hind III (5'Δ1692, 3'Δ1766) und Spe I (5'Δ1611).
- Fig. 3.:: Hydrophobozitäts Blot der computerabgeleiteten Proteinsequenz des ETAA16 Antigens nach der Analysemethode von Kyte und Doolittle: durchgezogene Linie symbolisiert die Basislinie, die gestrichelte Linie gibt den "cut off " für membran-assoziierte Proteinbereiche an und der Pfeil markiert eine potentiell membranständige Region des Proteins.
- Fig. 4.:: Epitopkartierung des ETAA 16 Antigens: Die mit Hpa I/Sac I behandelten 3'-Deletionsklone von ETAA16 cDNA und die Vollängenklone 6.1.1 und 3.1.1 (3'Δ150 bp, 5'Δ54) wurden über ein 0,7 % Agarosegel getrennt (a) und im Western Blot eines 10 % SDS PAGES mit dem Antikörper 16 umgesetzt (c). Für einen Kolonie-Immunoscreen wurden die cDNA Klone in XL-1 blue *E. coli* transformiert, die entsprechenden Übernachtkulturen auf LB-Platten ausgestrichen, auf Nitrocellulosemembranen übertragen und mit einem Hybridomaüberstand des Antikörpers 16 inkubiert (b).
- Fig. 5.:: Darstellung der Oberflächenexpression der ETAA16 cDNA Klone nach transienter Transfektion in den Zellen der humanen Zellinie COS-7 nach ihrer Inkubation mit dem Antikörper 16 und Detektion der Antikörper-Bindung im Durchflußzytometer.

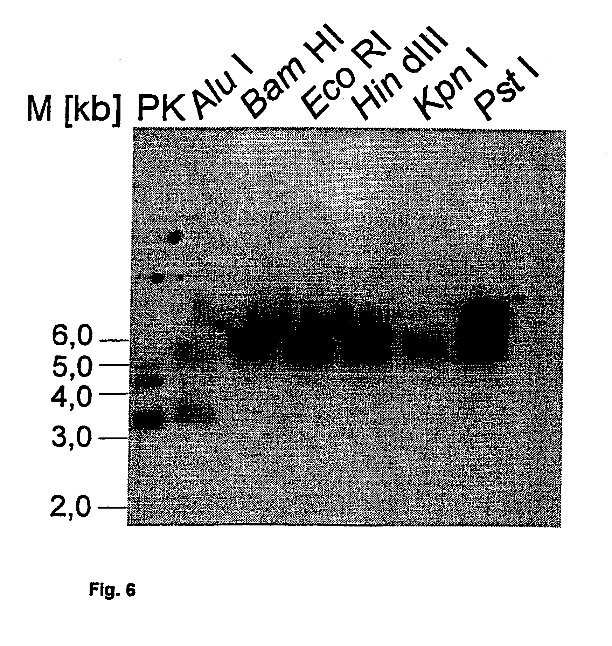

- Fig. 6.:: Darstellung der intrazellulären Expression der ETAA16 cDNA Klone nach transienter Transfektion in den Zellen der humanen zellinie COS-7-Zellen nach Fixierung der transfektierten Zellen, Inkubation mit dem Antikörper 16 und Detektion der Antikörper-Bindung im Durchflußzytometer.

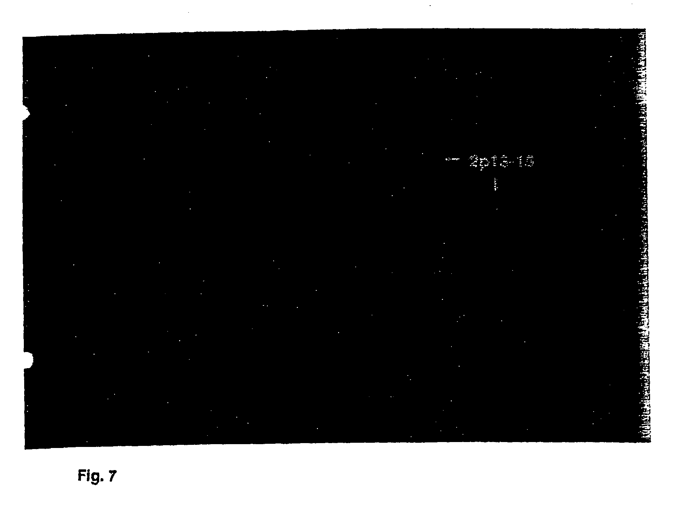

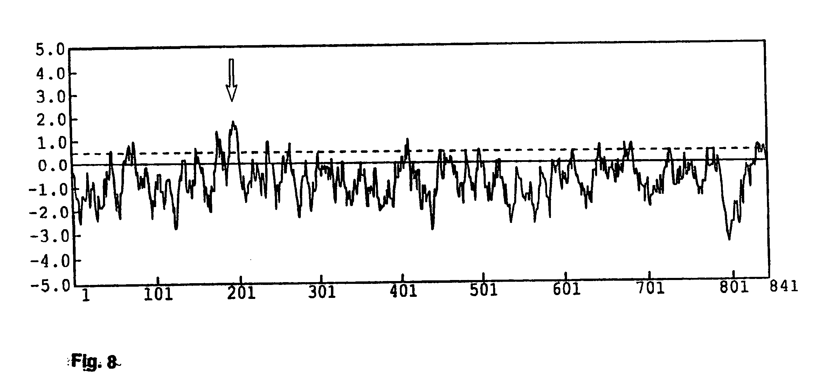

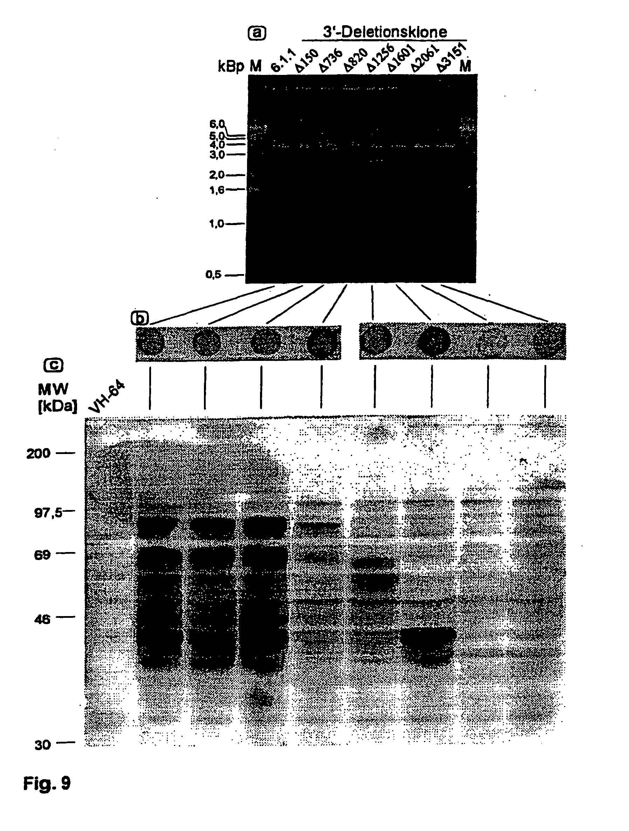

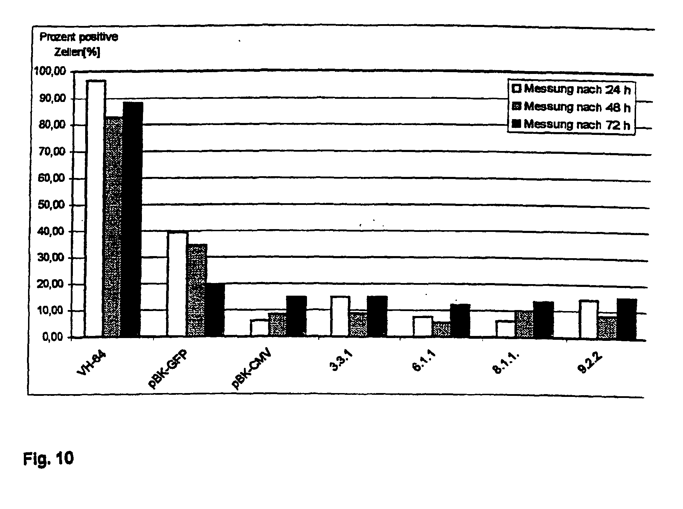

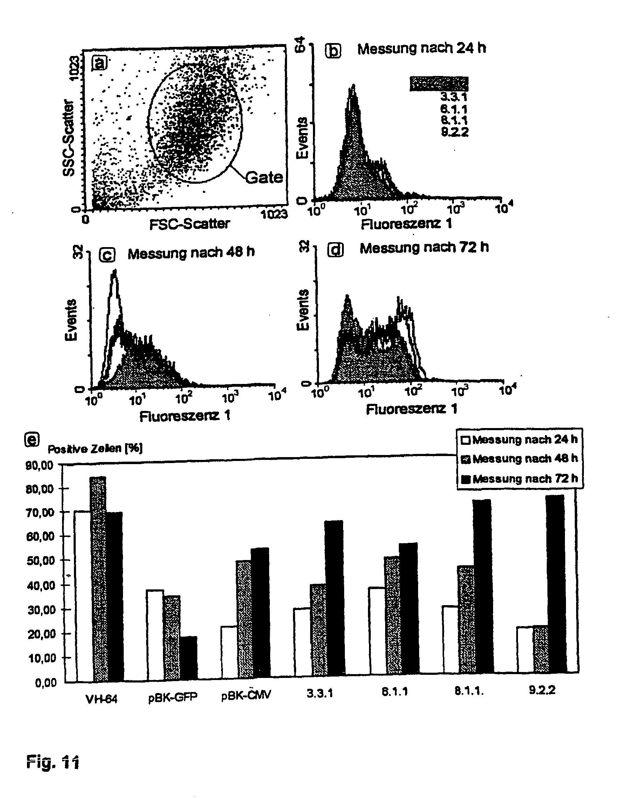

- Fig. 7.:: ⁵¹Cr-Freisetzungsassay von nicht adhärenten Lymphozyten (NAL) und Lymphokin aktivierten Killerzellen (LAK) gegen Zellen der myeloischen Leukämie Zellinie K562 und der ET-Zellinie RD-ES mit vier verschiedenen Effektor/Target-Konzentrationen (100:1; 33:1; 11:1; 4:1) in vier verschiedenen Ansätzen (kein Zusatz; Antikörper 16 aus einem Hybridome-Überstand; Phytohämagglutinin (PHA); Antikörper 16+PHA).

Der Begriff "cDNA" wird hier verwendet als DNA-Kopie einer mRNA-Vorlage. Unter einem "cDNA-Klon" wird die cDNA summiert, die aus der Klonierung des identischen cDNA-Fragments entstanden ist. Ein "Deletionsklon" ist ein cDNA-Klon, der eine Nukleinsäuresequenz definierter Länge seiner ursprünglichen cDNA-Sequenz verloren hat. Dies kann aufgrund einer Behandlung mit einer Exonuklease geschehen. Dagegen ist ein "Vollängenklon" definiert als ein cDNA-Klon der ursprünglichen Länge ohne nachfolgenden Verlust eines DNA-Fragments. Als "homologe Sequenzen" werden diejenigen Sequenzen verstanden, die eine ähnliche Sequenz aufweisen.

Als ein "Antigen" wird jedes Molekül verstanden, das eine spezifische Immunantwort auslösen bzw. mit Komponenten einer bereits ablaufenden Immunantwort reagieren kann. Es kann sowohl eine Antikörper vermittelte oder auch T-Zell spezifische Antwort induzieren. Ein "tumorspezifisches Antigen" (TSA) charakterisiert als neuartiges Antigen die Tumorzelle in Abgrenzung zu einer gesunden Gewebezelle. Dagegen bezeichnet ein "tumorassoziiertes Antigen" (TAA) ein Antigen, das überhaupt nicht oder aber in nur kleinen Mengen von einer gesunden Zelle exprimiert wird, bei Tumorzellen aber verstärkt vorkommt.

Der Begriff der "Immundiagnose" ist hier verwendet als eine Bezeichnung für alle *in vitro* oder *in vivo* Verfahren und Techniken zum Nachweis, der Identifikation und der Lokalisation von Tumoren. Die "Immuntherapie" umfaßt als Sammelbegriff sowohl alle Formen der aktiven, passiven, spezifischen und unspezifischen kurativen Behandlungen von Tumoren als auch der Prävention und Etablierung ihrer Enstehung.

Ein "Antikörper" wird hier definiert als ein Molekül, das als Reaktion auf den Kontakt mit einem Antigen gebildet oder unter Zuhilfenahme eines Antigens erzeugt wird, und an dieses Antigen spezifisch binden kann. Dabei ist das "Epitop" oder die "Antikörperbindungsstelle" eine Determinante des Antigens, an die der Antikörper oder Teile desselben binden können.

Ein Oligonukleotid bezeichnet ein Molekül aus zwei oder mehreren Desoxyribonukleotiden oder Ribonukleotiden. Ein "Primer" wird hier definiert als ein natürliches oder synthetisches Oligonukleotid, das eine Nukleinsäuresynthese initiieren kann und vorzugsweise einzelsträngig ist. Es kann aber auch doppelsträngig sein.

Zur Identifizierung und Isolierung der ETAA16 cDNA wurde eine cDNA Expressionsbibliothek der Ewing Tumor Zellinie VH-64 in dem Derivat des λ ZAP verwendet, welcher 5' vom lacZ Promoter zusätzlich den CMV Promoter für eine eukaryotische Expression trägt. Diese Konstruktion ist genauer bei Short et al., Nucleic. Acids. Res. 16: 7583 (1983) beschrieben. Die cDNA Expressionsbibliothek hatte einen Primärphagentiter von ∼5x10⁵ und einen Anteil von nicht-rekombinanten λ Phagen von ca. 2%. Die Integrität der cDNA Expressionsbibliothek wurde durch die Größenbestimmung der cDNA Insertionen von 26 zufällig ausgewählten Klonen analysiert.

Die isolierten Phagen wurden durch die *in vivo* Excision in das Phagemid pBK-CMV überführt und die cDNA Insertionen durch die Inkubation mit Eco RI und XhoI ausgeschnitten. Die Größe der klonierten cDNA wurde in einem 1% TBE-Agarosegel bestimmt. Lediglich 7% der analysierten Klone trugen eine cDNA, die kleiner als 1 kb war. Die Primär-cDNA Bibliothek wurde mit 3-4x10⁴ pfu pro 150 mm² NZY-Agarplatte amplifiziert. Die amplifizierte VH-64 cDNA Expressionsbibliothek besaß einen Titer von ∼1,5x10⁹ pfu/ml.

Zur Isolierung der von den ET spezifischen Antikörpern definierten Antigenen wurde die cDNA Expressionsbibliothek der Zelllinie VH-64 mit dem Pool aus der Durchflußzytometrie von Hybridomaüberständen der ET spezifischen Antikörper aus Balb/c Mäusen durchsucht, dabei wurden 5x10⁵ Phagen (Titer der Primärbibliothek) ausplattiert. Die Gewinnung dieser Antikörper kann bei Shi et. al., Cancer Immunol. Immunother. 38: 208-213 (1994) nachgelesen werden.Nachdem Primärimmunoscreen wurden 27 Klone auf den doppelt-gezogenen Filtern identifiziert. Im nachfolgenden Rescreen bestätigten sich 9 Klone als tatsächlich positiv, von denen sich 5 Klone (2.1.1, 4.3.1, 5.1.1, 5.2.1, 10.2.1) durch ihre Detektion vom 2. Antikörper Ziege-α-Maus (H+L) konjugiert mit alkalischer Phosphatase als falsch-positiv herausstellten. Die verbleibenden 4 Klone 3.3.1, 6.1.1, 8.1.1, 9.2.2 konnten dem ET spezifischen Antikörper 16 (SN58/1857.15.30) zugeordnet werden. Dieser Antikörper wird für die weiteren Schritte der Isolierung der ETAA16cDNA und des ETAA16 Antigens verwendet und als Antikörper 16 bezeichnet.

Die isolierten Klone wurden durch die *in vivo* Excision in die Phagemid-Form pBK-CMV des λ ZAP-Express™ überführt und die cDNA-Insertionen durch die Inkubation mit *Eco* RI und *Xho*I aus dem Vektor ausgeschnitten. Die Größe der klonierten cDNAs wurde in einem 0,8% TBE-Agarosegel bestimmt.

Die Klone, deren Transkriptionsprodukte der Antikörper 16 erkannte, tragen eine ∼3,5 kb große cDNA mit einer 350 bp langen Eco RI Erkennungssequenz. Lediglich der Klon 3.3.1 zeigt ein cDNA Insertion von ca. 3,3 kb und weist in diesem Fragment eine Deletion von ca. 150 bp auf.

Die drei 3,5 kb Klone 6.1.1, 8.1.1, 9.2.2 und der 3,3 kb Klon 3.3.1 werden als ETAA16 cDNA Klone, und die entsprechenden Transkripte als ETAA16 Antigen bezeichnet. Alle Klone wurden vom 5'- und 3'-Ende ansequenziert. Demnach sind die drei Klone 6.1.1, 8.1.1, 9.2.2 mit der 3,5 kb Insertion identisch. Der ETAA16 Klon 3.3.1 besitzt dagegen eine Deletion von 54 bp am 5'-Ende und eine Deletion von 160 bp am 3'-Ende.

Zur Erstellung der vollständigen Sequenz wurden vom Klon 6.1.1 mit Hilfe der Exonuklease III sowohl 5'- als auch 3'-Deletionsklone erzeugt. Das pBK-CMV Rückgrat wurde bei den 5' Deletionsklonen durch Inkubation mit dem Restriktionsenzym *Bss* HII und anschließendem Auffüllen der überhängenden Ende mit α-thio Phosphat dNTPs geschützt, während die 3'-Deletionsklone durch das Öffnen des Phagemidklons mit den Restriktionsenzymen *Kpn* I bzw. *Bst* XI, die geschützten 3'-Überhänge produzierten.

Zusätzlich wurden die intern auftretenden Restriktionsschnittstellen der Restriktionsenzyme aus der *Multi Cloning Site* (MCS) ebenfalls dazu verwendet, Deletionsklone zu subklonieren. Dazu wurde der Klon 6.1.1 mit dem jeweiligen Restriktionsenzym geöffnet, das pBK-CMV Rückgrat mit dem restlichen Teil des Klons 6.1.1 im Agarosegel gereinigt und religiert. Folgende Restriktionsenzyme wurden dazu verwendet: *Sma* I (3'Δ3145), *Eco* RI (5'Δ471), *Hin*dIII (5'Δ1692, 3'Δ1766), *Spe* I (5'Δ1611). Die *Hin*dIII Schnittstelle geht durch die Klonierung der cDNA in die *Eco* RI und *Xho*I Erkennungssequenzen verloren, deshalb wurden die *Hin*dIII Deletionsklone zusätzliche mit *Bam* HI für die 5'-Deletion und *Xho* I für die 3'-Deletion inkubiert und nach einer Auffüllreaktion religiert.

Die vollständige Sequenz des ETAA16 Antigens beträgt einschließlich des Eco RI Adapters und der *Xho* I Erkennungssequenz 3457 bp (siehe Seq Id No:1). Es wurde mit der ALFwin.1.1 Software prozessiert und mit Hilfe des DNASIS Programms von Hitachi ausgewertet:. Demnach liegt das offene Leseraster (ORF) im ersten Leserahmen: Das Startcodon befindet sich an Position 403 und das Stopcodon an Position 2925. Das Startcodon liegt innerhalb eines guten Kontexts zur Kozak Sequenz, die die Initiation der Translation, ein A in Position -3 und ein G in Position+4 begünstigt. Neben diesem ORF treten in der 5`-UTR weitere kurze ORFs in allen 3 Leserastem auf: 1: 166-258; 2.: 221-292 und 3: 147-356. Dies besitzen aber alle vor dem eigentlichen Startcodon von ETAA16 ein Stopcodon. Diese Startcodons tragen nicht die Kozak Sequenz, so daß der Initiationsapparat der Translation diese übergehen kann.

Im 3'-UTR Bereich befinden sich drei potentielle Polyadenylierungssignale in den Postionen 3057, 3296 und 3408 bp. Zusätzlich läßt sich ein AU-rich Element (ARE) an der Position 3053 bp vor der ersten potentiellen Polyadenylierungsstelle identifizieren. Der 402 bp umfassende 5'-UTR Bereich des ETAA16 besitzt einen G/C-Gehalt von 62,18 %, während die 3'-UTR Region lediglich einen G/C-Gehalt von 26,98% erreicht. Das offene Leseraster besitzt einen G/C-Gehalt von 34,4 % und liefert ein Protein von 841 AS mit einem potentiellen Molekulargewicht von 94427,03 Da.

Der Homologievergleich der die cDNA-Sequenz mit bereits bekannten EST-Sequenzen wurde mit dem Basic Local Alignemt Search Tool (BLAST) vom National Center for Biotechnology Information (NCBI) und der FastA Analyse vom European Bioinformatics Institute (EBI) durchgeführt.

Es wurden Homologien zu folgenden humanen EST-Sequenzen gefunden: Eine 197 bp lange embryonale EST Sequenz (Accession AA330059) aus einer von einem 12 Wochen alten Embryo erstellten cDNA Expressionsbibliothek ist zu 100% homolog für den 5'-Bereich der 594-790 bp ETAA16cDNA. Im 3'-Bereich wurde eine 100% homologe Sequenz von 78 bp gefunden (3241-3319 bp), bei der es sich um eine Gen-Signatur aus einer adulten humanen 3'-orientierten cDNA Expressionsbibliothek handelt (Accession C21290).

Im 3'-Bereich treten drei homologe Sequenzen aus Expressionsbibliotheken fötaler Gewebe auf. Eine 665 bp umfassende EST Sequenz aus einer fötalen lungenspezifischen cDNA Expressionsbibliothek (Accession W16645) weist eine Homologie von 97,2% über 462 bp (2605-3074 bp) zur ETAA16 cDNA auf. Daneben gibt eine EST Sequenz aus fötalem Lungengewebe (571 bp, Accession W16643) mit einer Homologie von 96,6 % über 495 bp der ETAA16 cDNA. Eine dritte 324 bp lange unabhängige homologe fötale EST Sequenz aus einer Gehim cDNA Bibliothek (Accession D60196) ist in dem Bereich von 2793 bis 3119 bp zu 93,6% mit der ETAA16 cDNA homolog.

Zusätzlich wurden fünf homologe Sequenzen aus differenzierten Geweben gefunden. Aus einer Plazenta cDNA Bibliothek wurden zwei Klone mit nahezu identischer Sequenz isoliert: Die 343 bp lange Plazenta EST Sequenz mit der Accesion R78670 ist zu 99,4% homolog zu dem ETAA16 cDNA Bereich von 2457 bis 2799 bp. Die zweite Plazenta EST Sequenz (Accession R32610) aus derselben cDNA Bibliothek weist eine Länge von 2822 bp und eine Homologie von 96,2% auf. Eine 232 bp lange EST Sequenz aus einer Aorta-spezifschen Expressionsbibliothek (Accession D62095) besitzt zu der ETAA16 cDNA in dem Bereich zwischen 2929 und 3160 eine Homologie von 91,4 %. Die dritte unabhängig homologe EST Sequenz wurde aus einer humanen cDNA Expressionsbibliothek von Tonsillar Zellen isoliert (Accession AA829856). Diese 318 bp lange EST Sequenz weist eine Homologie von 97,6% über 297 bp auf, die im potentiellen 5' untranslatierten Bereich von ETAA16 cDNA von 74 bis 370 bp liegt.

Neben den homologen humanen EST-Sequenzen ergaben die FastA und BLAST Untersuchungen Homologien zu nicht humanen Sequenzen. Eine cDNA aus einer Expressionsbibliothek von *Bos taurus* zeigt die höchsten Homologien zu ETAA16 cDNA. Es handelt sich dabei um eine cDNA, die für ein zytoplasmatisches Protein aus den Junction Plaques von Sertoli Zellen und Hepatozyten (Accession Y08459) kodiert.

Diese cDNA aus *Bos taurus* besitzt mehrere Blöcke von homologen Sequenzen. Der erste Block liegt im 5'-Bereich der ETAA16 cDNA und besitzt über 1323 bp, die in der ETAA16 cDNA dem Bereich von 45-1359 bp entsprechen, eine Homologie von 83,7. Es liegen drei weitere Bereiche mit längeren homologen Sequenzen vor: 85,2% über 345 bp (entsprechen bei der ETAA16 DNA dem Abschnitt von 1735-2079 bp), 83,2% über 339 bp (entsprechen bei der ETAA16 cDNA dem Abschnitt von 2192-3531 bp) und 84,5% über 290 bp (entsprechen bei der ETAA16 cDNA dem Abschnitt von 2611-2900 bp). Zusätzlich treten kürzere homologe Blöcke auf: Die ersten vier liegen im Bereich von 1376-1682 bp mit einer Länge von 19 bis 118 bp und Homologien zwischen 84 und 94%. Im 3'-Bereich von ETAA16 cDNA von 2916-3284 bp treten acht weitere Bereiche mit Homologien von 84% bis 94% auf, dabei sind die homologen Bereiche zwischen 17 und 43 bp lang.

Neben dieser *Bo*s *taurus* cDNA Sequenz wurden durch die FastA Homologievergleiche einige EST Sequenzen aus cDNA Expressionsbibliotheken aus Mausgeweben gefunden. Zwei homologe Sequenzen wurden aus cDNA Expressionsbibliotheken von CD4 positiven T-Zellen isoliert, dabei weist die erste T-Zell spezfische-EST Sequenz (Accession AA666753) eine Homologie von 75,3% über 392 bp (712-1098 bp) auf. Die zweite 421 bp lange T-Zell spezifische EST Sequenz (Accession AA666724) zeigt Homologien zum mittleren Teil der ETAA16 cDNA und zwar im Bereich von 1755 bis 2073 bp mit einer Homologie von 65,8% über 313 bp. In diesem Bereich wurden zudem eine homologe cDNA Sequenz aus einer Expressionsbibliothek aus der Haut einer 11 Wochen alten weiblichen Maus, isoliert (Accession AA756745). Die cDNA Sequenz zeigt eine Homologie von 69,5% über 357 bp (1703-2059 bp).

Es wurden weitere homologe EST Sequenzen aus cDNA Expressionsbibliotheken verschiedener embryonaler Entwicklungsstadien gefunden: Eine EST Sequenz aus der Expressionsbibliothek eines 7,5 Tage alten Gastrulastadiums zeigt für den 5'-Bereich von 891-1354 bp eine Homologie von 75,9% über 463 bp. Die restlichen drei EST Sequenzen aus embryonalen Geweben unterschiedlichster Entwicklungsstadien sind homolog zum 3'-Bereich von ETAA16 cDNA. Die längste EST Sequenz (508 bp) wurde aus der Expressionsbibliothek eines 2-Zellstadium Mausembryos ermittelt und besitzt über 272 bp eine Homologie von 73,5% (Accession AA473938). In dem ETAA16 cDNA Abschnitt von 2953-3089 bp sind zwei weitere EST Sequenzen aus Expressionsbibliotheken von 7,5 und 5,5 alten embryonalen Gewebe zu 85,71% (82 bp) bzw. zu 82,86% (140 bp) homolog. (Accession AA139242 und AA396877).

Aus den Homologievergleichen ist abzuleiten, daß keine vollständige humane homologe cDNA bisher beschrieben wurde. Durch Hybridisierungsversuche und eine FISH-Analyse (Fluorescence In Situ Hybridisation) wurde die Annahme bestätigt, daß es sich um ein humanes "single-copy" Gen handelt:

Der Southern Blot in Figur 1 zeigt, daß die ETAA16 cDNA-Sonde von 501 bp, welche aus dem 3'-Bereich durch einen Doppelansatz *Dra* I/*Dra* III mit anschließender Gelaufreinigung isoliert und markiert wurde, mit genomischen DNA-Fragmenten einer Größe von -6 kb nach der Inkubation mit Hexanukleotid erkennenden Restriktionsenzymen (*Bam* HI, *Eco* RI, *Hin* dIII, *Kpn* I, *Pst* I) hybridisiert.

Lediglich nach der Inkubation der genomischen DNA mit *Alu* I, welches eine Tetranukleotiderkennungssequenz besitzt, hybridisiert die Sonde mit zwei Fragmenten von 3,5 und 5,5 kb Größe, obwohl die Sonde keine interne *Alu* I Erkennungssequenzen besitzt. Dies zeigt, daß die Sonde von verschiedenen Exons kodiert wird und im Intron Erkennungssequenzen für *Alu* I existieren. Als Positivkontrolle wurde der Klon 6.1.1 nach der Doppelinkubation mit *Bam* HI und *Xho* I verwendet, wobei die Sonde mit der erwarteten kompletten cDNA von 3,5 kb hybridisiert. Die 4,5 und 5,0 kb Banden innerhalb der Positivkontrolle erklären sich durch eine Kreuzhybridisierung mit dem Rückgrad des Vektors (4,5 kb) bzw. durch die Hybridisierung mit unvollständig geschnittenem ETAA16 Klon 6.1.1.

Die FISH-Analyse ergab für DNA Sequenz des ETAA16 Antigens eine Lokalisation auf dem Chromosom 2p13-15 (Figur 2). In die FISH Analyse wurden Subklone der ETAA16 cDNA, die durch die internen Restriktionsschnittstellen von Restriktionsenzymen aus der MCS (*Sma* I (3'Δ3145), *Eco* RI (5'Δ471), *Hin*dIII (5'Δ1692, 3'Δ1766), *Spe* I (5'Δ1611)) hergestellt worden sind, eingesetzt.

Die cDNA Sequenz des ETAA16 gemäß SEQ. ID. N.1 dient als Matrize zur Herstellung von Oligonukleotiden, die in der spezifische Detektion der ETAA16 mRNA eingesetzt werden. Diese Oligonukleotide dienen als Primer oder zur Herstellung von Primem und Oligonukleotiden, die vorzugsweise in die Polymerasdekettenreaktion beispielsweise zur Identifikation der spezifischen ETAA16 cDNA Transkripte eingesetzt werden. Die Primer und Oligonukleotide werden anhand der cDNA Sequenz und des ETAA16 Antigens erstellt, damit diese exonübergreifend sind. Die Aminosäuresequenz nach Figur 5 besitzt einen transmembranen Bereich (siehe Figur 3), so daß dieser Sequenzbereich von verschiedenen Exons kodiert wird. Die Primer oder Oligonukleotide werden entsprechend aus diesem Sequenzbereich gewählt, die diese Aminosäuresequenz codieren.

Die Isolierung des genomischen Klons von ETAA16 vom Chromosom 2p13-15 ermöglicht den Einsatz weiterer Primer und Oligonukleotide. Diese werden unter anderem für die diagnostische Polymerasekettenreaktion (PCR) eingesetzt, die sowohl mit RNA aus Biopsien als auch aus peripherem Blut oder Knochenmark durchgeführt wird. Zusätzlich wird vorzugsweise die *in situ*-PCR verwendet, die eine Reinigung der RNA umgeht und direkt auf Gewebeschnitten durchgeführt wird. Die Methodik der PCR wird nach Standard Protokollen durchgeführt, wobei sich lediglich die Wahl der Primer oder Oligonukleotide sich aus der ETAA16 cDNA Sequenz ergibt. Nähere Einzelheiten zu der Methodik siehe auch McPherson et al., PCR: A practical approach, Oxford University Press (1991).

Der genomische Klon des ETAA16 Gens wird unter Verwendung der ETAA16 cDNA isoliert, indem eine humane genomische Bibliothek, die in Cosmid Vektoren erstellt wurde, mit Teilen der ETAA16 cDNA hybridisiert wird. Dazu werden Fragmente der ETAA16 cDNA nach der Standard Methode von Feinberg und Vogelstein radioaktiv markiert. Sieh dazu auch Feinberg und Vogelstein, Analytical Biochemistry 178: 6-13 (1983). Diese Fragmente werden mit der genomischen Bibliothek, die auf Nitrocellulosemembranen übertragen wurde, hybridisiert. Die positiven Klone werden nach Standardprotokollen isoliert, gereinigt und analysiert. Einzelheiten dazu bei Sambrook et al. A laboratory Manual. Chapter 9 2nd edition (1989). Der klonierte genomische Klon wird sequenziert, und seine Exonstruktur mit Hilfe der ETAA16 cDNA analysiert. Diese dient der exonübergreifenden Positionierung der Primer und Oligonukleotide insbesondere für die PCR.

Mit der ETAA16 cDNA gemäß SEQ. ID. Nr.1, die für das komplette zelluläre Protein ETAA16 codiert, ist die Aminosäuresequenz nach den Grundlagen des genetischen Codes definiert. Sie ist ebenso im Sequenzprotokoll dargestellt. Aus der Aminosäuresequenz können Peptide z.B, mit Hilfe eines im Handel erhältlichen Peptidsynthetisiergerätes hergestellt werden. Siehe dazu auch Rudensky, Nature 353: 622 - 627. Die Peptide können als überlappende Sequenzen produziert und zur Herstellung monoklonaler Antikörper eingesetzt werden. Sie lassen sich chemisch durch Tripalmitoyl-S-glycerylcystein modifizieren. Dieses Verfahren steigert ihre Immunogenität in *vivo.*

Die erfolgreiche Herstellung monoklonaler Antikörper gegen Peptide, die aus einer Aminosäuresequenz eines Proteins theoretisch abgeleitet und chemisch hergestellt wurden, ist in R. Spohn, B-, Th- und CTL-Epitope des HIV-1 Nef-Proteins, Dissertation der Fakultät für Chemie und Pharmazie, Universität Tübingen (1993) detailliert nachzulesen.

Das von der ETAA16 cDNA codierte Polypeptid besteht aus 841 Aminosäuren mit einem errechneten Molekulargewicht von 94427,03 Da. Der berechnete isoelektrische Punkt liegt bei pl 6,86. Das Polypeptid ist reich an Serin (11,89%), Lysin (9,51%), Threonin (8,44%), Asparagin (8,32%), Leucin (7,13%), Glutaminsäure (6,77%) und Isoleucin (6,42%).

Die translatierte Sequenz von ETAA16 cDNA wurde nach der Methode von Kyte-Doolittle einem Hydrophobizitäts Blot mit einem Fenster von 10 Aminosäuren unterzogen (Figur 3). Demnach weist das ETAA16 Antigen einen potentiell membranständigen Bereich der Aminosäuren 186 bis 203 auf. Es liegen weitere 10 kurze hydrophobe Bereiche mit einem Wert größer als +0,5 vor. Diese stellen eher interne Domänen des ETAA16 Antigens dar, da diese Regionen eine geringere Hydrophobizität erreichen als der potentiell membranständige Bereich. Es wird deutlich, daß viele Abschnitte des ETAA16 Antigens einen Wert kleiner als -0,5 besitzen.

Zur Epitopkartierung des ETAA16 Antigens wurden die Vollängenklone 6.1.1 und 3.1.1 und die 3'-Deletionsklone im Kolonie-Immunoscreen und im Western Blot (Figur 4) untersucht. Zur ihrer Längenbestimmung wurden diese im Doppelansatz mit *Hpa* I/*Sac* I inkubiert, da die 3'-Deletionsklone durch Öffnen des Vektors mit *Kpn* I hergestellt und somit alle Restriktionserkennungssequenzen der MCS 3' der ETAA16 cDNA (*Xho* I-*Kpn* I) verloren wurden. Daher wurden die Subklone mit *Hpa* I ausgeschnitten. Daraus folgt, daß sich die eigentliche Größe der Subklone um 427 bp verlängerte, weil *Hpa* I 3' von der cDNA des GFP in der SV40 Polyadenylierungsstelle des pBK-CMV schneidet.

Die beiden Vollängenklone 6.1.1 und 3.3.1, der eine 150 bp Deletion im 3'-Bereich und eine 54 bp Deletion im 5'-Bereich aufweist, wurden durch den Antkörper 16 markiert. Lediglich die 3'-Deletionsklone Δ2061 und Δ3151, die durch die Subklonierung des *Sma* I Fragmentes entstanden sind, kodieren nicht mehr für das Epitop des Antikörpers. Das Epitop wird von einem Fragment kodiert, daß zwischen dem 1396 und 1856 bp liegt und für die Aminosäuren 332-485 kodiert. Da die Epitope von Proteinen und Polypeptiden häufig eine Länge von etwa 15 Aminosäuren besitzen, sind in diesem 115 Aminosäuren umfassenden Peptid des ETAA16 Antigens vermutlich Bereiche vorhanden, die nicht für dieses Epitop benötigt werden. Möglicherweise bilden sie weitere Epitope.

Der Western Blot der beiden Vollängenklone und der 3`-Deletionsklone bestätigt die Annahme, daß ab Position Δ2061 das Epitop deletiert wurde.

Das in *E.coli* exprimierte ETAA16 Antigen besitzt ein Molekulargewicht von -90 kDa und weicht damit von der berechneten Größe um 5 kDa ab. Dies Phänomen kann durch die SDS-PAGE hervorgerufen werden, bei der Molekulargewichtsbestimmungen von Proteinen aufgrund von noch vorhandenen Sekundärstrukturen von dem berechneten Molekulargewicht häufig abweichen. Die detektierten Proteine mit niedrigerem Molekulargewicht sind auf Degradationprodukte des ETAA16 Antigens zurückzuführen, die besonders bei eukaryotischen Proteinen mit transmembran Domänen auftreten, sofern diese nicht als Fusionprotein exprimiert werden. Dies geht auf Ausführungen von Mount, Ann Rev. Genetics 14: 279-319 (1980), zurück. Der 3'-Deletionsklon Δ820 ist der erste Klon, der ein geringfühgig kleineres Molekulargewicht als die Vollängenklone aufweist, da dieser aufgrund seiner 288 bp Deletion des translatierten Bereichs keine 3'-untranslatierte Region trägt. Der Δ1256 Deletionsklon besitzt ein Molekulargewicht von 55-63 kDa und der Subklon mit der Deletion von Δ1601 bp kodiert für ein Protein von etwa 43 kDa.

Die Epitopkartierung macht deutlich, daß der Antikörper 16 die Klone im prokaryotischen System erkennt. Transiente Transfektionsexperimente zeigen weiterhin die Oberflächenexpression des von ETAA16 cDNA codierten Antigens auf transfektierten Zellen der humanen Zellinie COS-7 Zellen (Figur 5).

Die Transfektionseffizienz der COS-7 Zellen wurde mit dem Phagemid pBK-GFP in der Durchflußzytometrie detektiert, dabei wurde das Phagemid pBK-CMV aus der VH-64 cDNA Expressionsbibliothek anhand der funktionsfähigen β-Galactosidase isoliert. Das Green Fluoreszent Protein (GFP) wurde aus dem pEGFP-1 isoliert und in die Bam HI/*Xba* I Schnittstellen kloniert, so daß das GFP sowohl unter der Kontrolle des prokaryontischen lacZ als auch des eukaryotischen CMV Promoters steht. Es konnten Transfektionseffizenzen von fast 40% nach 24 h erreicht werden.

In die Durchflußzytometrie wurde die Zellinie VH-64 als Positivkontrolle und die mit dem pBK-CMV transfektierten COS-7 Zellen als Negativkontrolle für die Antikörper-Bindung eingesetzt: Die Klone 3.3.1 und 9.2.2 waren nach 24 h auf der Oberfläche mit 14,49% bzw. 14,31% der Zellen nachweisbar. Die Klone 6.1.1 und 8.1.1 zeigen ähnliche Ergebnisse wie die Negativkontrolle.

Die Expression des von ETAA16 cDNA codierten Antigens wurde intrazellulär in den transfizierten COS-7 Zellen im Durchflußzytometer untersucht (Figur 6). Dazu wurde die transfektierten COS-7 Zellen fixierte und ebenfalls mit dem Antikörper 16 markiert.

Die verschiedenen Klone der ETAA16 cDNA wurden nach 24 h schwach in den transfizierten COS-7 Zellen exprimiert, lediglich der Klon 9.2.2 exprimierte das Antigen zu diesem Zeitpunkt nicht. Nach 48 h erschien die Expression der ETAA16 cDNA Klone heterogen, da die Negativkontrolle mit 48,06% eine hohe Kreuzreatkivität mit dem Antikörper 16 besitzt. Dagegen ist die Expression der ETAA16 cDNA nach 72 h von allen 4 Konen nachweisbar, allerdings wurden die Klone 8.1.1 und 9.2.2 mit 71,14 bzw. 72,87% stärker exprimiert als die Klone 3.3.1 und 6.1.1 mit 63,31 bzw. 53,03%.

Der Antikörper 16 bindet zusätzlich an fixierte Zellen der ET Zellinie VH-64. Dies Bindung liegt mindestens bei 70% und ist somit geringer als die Oberflächenbindung des Antikörpers an der ET Zelllinie VH-64. Somit kann auch das fixierte Epitop vom Antikörper detektiert werden.

Blockierungsexperimente mit verschiedenen ET Zellinien zeigen die Funktion des ETAA16 Antigens bei der durch Natürliche Killerzellen (NK) vermittelten Zellyse.

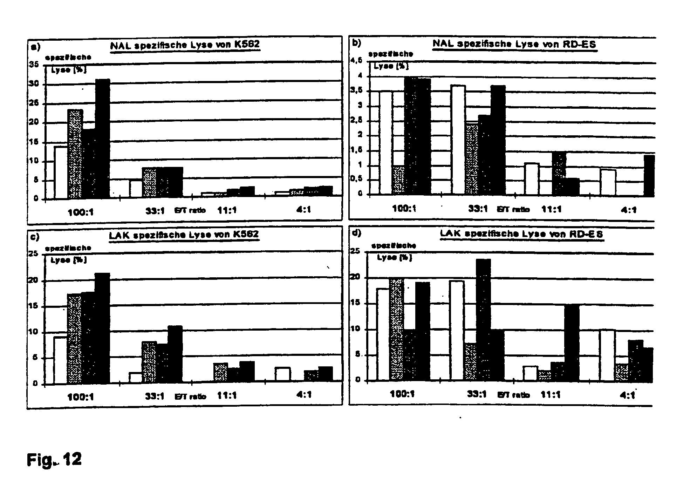

Die NK-spezifische Lyse wurde im Zytotoxizitätsassay mit der nichtadhärenten Lymphozyten-Fraktion (NAL) verschiedener Spender ermittelt (Figur 12). Die Lymphokin aktivierten Zellen (LAK) wurden von denselben Spendern generiert. Mit einer etwa 3,5% spezifischen Lyse stellen die Zellen der Zellinie RD-ES ein deutlich schlechteres Target für die NK-Zellen dar als die myeloische Leukämie-Zellinie K562 mit 13% spezifischer Lyse. Die Präinkubation mit dem Antikörpers 16 führte zur inhibition der NK-spezifischen Zytotoxozität gegen die ET Zellinie RD-ES, die bei einem Effektor/Target-Verhältniss (E/T-Ratio) von 11:1 ausblieb. Mit einer 23,5% spezifischen Lyse wurde bei einem E/T-Verhältnis von 100:1 die Zytotoxizität gegen die Zellinie K562 durch den Antikörper 16 sogar verstärkt. Die NK-spezifische Zytotoxizität nahm mit niegrigerer Effektorzellzahl ab. Der Einfluß des Antikörpers 16 auf die Gesamtzytotoxizität, welche durch die Vernetzung der Effektorzelle mit der Targetzelle durch das Lektin Phytohämagglutinin (PHA) ermittelt wurde, war für die beiden verwendeten Zellinien unterschiedlich. Die NK-spezfische Gesamtlyse der Zelllinie K562 wurde durch den Antikörper 16 mit Ausnahme des E/T-Verhältnisses 100:1, bei der die Gesamtlyse um 12,7% gesteigert worden ist, kaum beeinflußt. Im Vergleich der verschiedenen E/T-Verhältnisse erscheint die Gesamtlyse der ET Zellinie RD-ES eher heterogen; allerdings zeigte sich bei einem E/T-Verhältnis von 100:1, bei dem die stärkste Inhibition der Zytotoxizität durch den Ak16 hervorgerufen wurde, kein Effekt auf die Gesamtlyse durch die Präinkubation mit dem Antikörpers 16.

Die Zytotoxizität von IL-2 aktivierten NK-Zellen (LAK-Zellen) war für die ET Zellinie RD-ES höher als für die K562 Zellinie. Die Zytotoxizität der LAK-Zellen gegenüber den Zellen der Zellinie K562 verändert sich bei der Zugabe des Antikörpers 16, von PHA und deren Kombination ähnlich wie die NK-Zell-spezifische Zytotoxizität. Mit Ausnahme des Effektor/Target-Verhältnisses von 100:1 inhibierte der Antikörper 16 auch die LAKspezifische Zytotoxizität gegen die ET Zellinie RD-ES.

Damit wird deutlich, daß die Ewing Tumorzellen der RD-ES Zellinie von den NK-Zellen zu einem geringeren Anteil lysiert werden, wenn der Antikörper 16 an das ETAA16 Antigen gebunden hat. Demnach inhibiert die Antikörper-Bindung die Zytotoxizität dieser Effektorzellen gegen diese Zellinie. Daraus folgt unmittelbar, daß das ETAA16 Antigen eine wesentliche Funktion in der Interaktion zwischen den Ewing Tumorzellen und den Natürlichen Killerzellen übernimmt. Dazu kann das ETAA 16 Antigen als Oberflächenprotein einerseits mit den aktivierenden Rezeptoren der NK-Zellen interagieren, oder als Adhäsionsmolekül die Interaktion zwischen der Effektorzelle und der Targetzelle erst ermöglichen. In beiden Fällen kann von einer regulativen Funktion des ETAA16 Antigens bei der NK-spezifischen Tumorlyse ausgegangen werden.

Die Isolierung und Charakterisierung der erfindungsgemäßen ETAA16 cDNA und des ETAA16 Antigens erfolgte mit folgenden Materialien und Methoden:

Es wurden folgende *E*. *coli* Stämme eingesetzt:

| ***E.coli* Stamm Genotyp** | |
|---|---|
| DH5a | F-, F80dlacZDM15, D(lacZYA-arg-F), U169, deoR, recA1, end A1, phoA, hsdR17(rk-, mk+), supE44, **I-,** thi-1, gyr A96, relA1 |
| VCS257 | Derivative of DP50 supF |
| XL-1 blue | recA1 endA1 gyrA96 thi-1 hsdR17 supE44 relA1 lac [F' proAB laclqZDM15 Tn10 (Tetr)] |
| XL-1 blue | D(mcrA)183 D(mcrCB-hsdSMR-mrr)173 endA1 supE44 thi-1 recA1 |
| MRF' | gyrA96 relA1 lac [F' proAB laclqZDM15 Tn10 (Tetr)] |
| XLOLR | D(mcrA)183 D(mcrCB-hsdSMR-mrr)173 endA1 thi-1 recA1 gyrA96 relA1 lac [F' proAB laclqZDM15 Tn10 (Tetr)] Su- Ir |

Es fanden folgende humane ET Zellinien Verwendung:

| **ET-Zellinien** | **Chromosomen translokationen** | **Typ des Fusions-produktes*** | **Klassifizierung der Pathologie** |
|---|---|---|---|
| A673 | n. d. | I | PNET |
| STA-ET-1 | 11/22 | I | PNET |
| STA-ET-2.1 | del(22)(q12) | VII | PNET |
| STA-ET-2.2 | del(22)(q12) | VII | PNET |
| STA-ET-5 | - | - | ES |
| GG-62 | del(22)(q12) | - | atypical ES |
| MA-ES | n. d. | | ES |
| NT-68^{§} | n. d. | II | PNET |
| RD-ES | del (22)(q12) | II | ES |
| RM-82 | del (22)(q12) | - | ES |
| SK-ES-1 | 11/22 | II | ES |
| VH-64 | 11/22 | II | ES |

| | | | |
|---|---|---|---|
| * Siehe für die Typisierung auch Dockhom et al., Virchows Arch. 425: 375-382(1994) | | | |

Die weiteren humanen Zellinien wurden eingesetzt:

| Zellinie | Zellart |
|---|---|
| COS-7 | SV40 transformierte Affenzelllinie |
| HeLa | Epitheliales Karzinom |
| K562 | Myeloische Leukämie |
| Monomac6 | Makrophagen Zelllinie |
| Polat S. | Keimzelltumor |
| T2 | T-Zelllinie, TAP negativ |

Die monoklonalen Antikörper gegen den Ewing Tumor, die hier verwendet wurden, sind von Shi et al., Cancer Immunol. Immunother. 38: 208-213 (1994) näher beschrieben:

| **Antikörper** | **Klon** |
|---|---|
| 1 | SN11 312.84.33/32 |
| 2 | SN11 813.92.23 |
| 3 | SN 11 944.73.47 |
| 4 | SN51 739.72.90 |
| 5 | SN54 1050.82.33 |
| 6 | SN54 1059.107.38 |
| 7 | SN55 159.50.12.15 |
| 8 | SN55 291.71.44 |
| 9 | SN55 303.45.16 |
| 10 | SN55 671.89.55/35 |
| 11 | SN55 697:89:23 |
| 12 | SN55 786.14.74134 |
| 13 | SN55 94.96.45 |
| 14 | SN58 1580.149.8 |
| 15 | SN58 162.28.11.37 |
| 16 | SN58 1857.15.30 |

Folgende Materialien wurden in der Durchflußzytometrie verwendet:

| | |
|---|---|
| FACScan | (Becton Dickinson) |
| FACS-Puffer: | PBS versetzt mit |
| | 0,1% (w/v) NaN₃ (Merck) |
| | 0,1% (w/v) BSA (Behring) |
| Propidiumjodid | (Sigma, Deisenhofen) |
| BFA-Fixans (pH 6,8): | 0,2 mg/ml Na₂HPO₄·2xH₂O |
| | 1,0 mg/ml KH₂PO₄ |
| | 45% (v/v) Aceton |
| | 9,25% (v/v) Formaldehyd |

Zur Analyse der Oberflächenexpression von membranständigen Zelloberflächenmolekülen wurden die Zellinien in 25 cm² kollagenbeschichteten Kulturflaschen über Nacht kultiviert. Die Zellen wurden mit 0,5 mM EDTA für 5-10 min bei 37°C abgelöst und anschließend 2x mit eiskaltem FACS-Puffer gewaschen. Intrazelluläre Antigene wurden zugänglich gemacht, indem die abglösten Zellen mit 500 µl BFA-Fixans unter ständigem Vortexen fixiert wurden. Anschlließend wurden die Zellen erneut in FACS-Puffer gewaschen und wie die lebenden Zellen weiter behandelt. Hier siehe auch Slaper-Cortenbach et al., Blood 72: 1639-1644 (1988).

Die Zellen wurden zu 2x10⁵ pro Ansatz aliquotiert und die Ansätze mit 20 µl des jeweiligen Hybridomaüberstandes des 1. Antikörpers (3-10 µg/ml) auf Eis im Dunkeln mindestens 30 min inkubiert. Neben den ET markierenden Antikörpern wurden als Positivkontrollen zwei Antikörper W6/32HL und B9.12.1 eingesetzt. Hierzu siehe auch Ziegler and Milstein Nature 279: 243 (1979) und Rebai and Malissen, Antigens 22: 107-117 (1983) eingesetzt. Die Ansätze wurden 2x mit 2-3 ml FACS-Puffer gewaschen und je für 3 min bei 4°C mit 225 g zentrifugiert. Der Antikörper-Antigen Komplex wurde mit einem farbstoffmarkierten α-Maus-2. Antikörper markiert. In diesem Fall wurden Fluoreszein-konjugierte-α-Maus-IgG F(ab')₂ Fragmente (Cappel, West Chester, USA) verwendet und diese Ansätze ebenfalls für mindestens 30 min auf Eis im Dunkeln inkubiert. Die Zellen wurden wie oben gewaschen und anschließend im Durchflußzytometer gemessen, dabei wurden tote Zellen durch Zugabe von 1µg pro Ansatz Propidiumjodid markiert, um diese bei der Auswertung ausschließen zu können.

Zur Bestimmung der zellvermittelten Zytotoxizität wurde der Cr⁵¹-Release Assay verwendet, bei dem die Targetzellen mit Na₂[⁵¹Cr]O₄ markiert werden. Die Target-Zellen wurden 3-4h mit 100 µgCi ⁵¹Cr bei 37°C und 5% CO₂ inkubiert und anschließend 3x mit PBS gewaschen.

Die Effektorzellen wurden aus peripherem Blut eines gesunden Spenders über einen Ficoll-Gradienten (1,077g/cm³ Seromed) isoliert, wodurch die mononukleären Zellen (PBMC) von den polymorphkemigen Zellen und den Erythrozyten getrennt werden. Das Heparinblut wurde vor dem Aufschichten auf den Ficoll-Gradienten mit 1 Vol PBS verdünnt. Der Gradient wurde für 20 min mit 839g bei Raumtemperatur zentrifugiert. Die Fraktion mit den mononukleären Zellen wurde 2x mit 10 Vol PBS gewaschen und bei 225g für 10 min pelletiert. Die Monozyten wurden durch eine einstündige Inkubation der mononukleären Zellen in 3 ml Kulturmedium/25cm² bei 37°C und 5% CO₂ entfernt.

LAK-Zellen wurden aus den isolierten PBMC durch Kultivierung mit 10³ IU/ml IL-2 in Kulturmedium für 72 h generiert.

Zur Untersuchung der Antikörperfunktion wurden die Zielzellen 15 min mit diesem bei 4°C präinkubiert. Anschließend wurden 50 µl der Effektorzellen zum jeweiligen Ansatz dazugegeben und für 4 h bzw. 16 h bei 37°C und 5% CO₂ inkubiert. Neben den Tumorzellinien wurde die K562 als Positivkontrolle eingesetzt. Die gesamtzytotoxische Aktivität wurde durch Inkubation mit 0,3% PHA-Medium ermittelt. Die Spontanlyse wurde nur mit den Targetzellen ermittelt und die maximale Freisetzung von ⁵¹Cr aus den Targetzellen wurde mit 2,5% Triton-X100 festgestellt. Nach den Inkubationszeiten wurden 25 µl aus den Ansätzen entnommen und auf Spoton-Filter aufgebracht. Die Filter wurden getrocknet, mit 10 ml Szintillationsflüssigkeit versetzt und die freigesetzte Radioaktivität im β-Counter (LKB) ermittelt.

Zur Isolierung genomischer DNA aus Zellinien wurde folgender Lysepuffer gewählt:

| | |
|---|---|
| Lysepuffer: | 10 mM Tris/HCl, pH 8,0 |
| | 100 mM NaCl |
| | 25 mM EDTA |
| | 0.5% SDS 0,1 mg/ml Proteinase K. |

Adhärente Zellinien wurden für die Isolierung genomischer DNA mittels Trypsin/EDTA für 5 min bei 37°C abgelöst und mit 10 Volumen PBS aufgefüllt. Die abgelösten Zellen oder Zellen aus Suspensionskulturen wurden bei 225g für 10 min bei 4°C zentrifugiert, 2x mit kaltem PBS gewaschen und die pelletierten Zellen in flüssigem Stickstoff schockgefroren bzw. direkt in Lysepuffer aufgenommen. 1x10⁸ Zellen wurden für 12-18 h mit 1 ml Lysepuffer inkubiert. Anschließend wurden die Nukleinsäuren mit 1 Vol TE gepuffertem Phenol, Phenol/Chloroform und Chloroform/Isoamylalkohol 49:1 extrahiert. Die RNA wurde mit 10 µg/ml RNase A für 3 h bei 37°C degradiert und die DNA durch Phenol/Chloroform und Chloroform/Isoamylalkohol 49:1 aufgereinigt. Die DNA wurde mit 2 M Ammonium-Acetat und 2 Vol Ethanol gefällt, mit 5000g für 5 min bei Raumtemperatur präzipitiert und mit 70% Ethanol gewaschen. Das Pellet wurde getrocknet, in 1 ml TE-Puffer/1x10⁷ aufgenommen und bei 4°C gelagert.

Die Isolierung der Gesamt-RNA lerfolgte nach der Single-Step Methode nach Chomzynski und Sacchi, Analytical Biochemistry 162: 156 (1987).

| | |
|---|---|
| Denaturierungslösung: | 4 M Guanidinium-isothiocyanat |
| | 25 mM Na-Citrat, pH 7,0 |
| | 0,1 M β-Mercaptoethanol |
| | 0,5% N-Lauroylsarkosyl |

Diese wurde als Stocklösung ohne β-Mereaptoethanol hergestellt, und erst kurz vor Gebrauch das β-Mercaptoethanol zugegeben.
2 M Na-Acetat, pH 4
Wasser-gesättigtes Phenol
Chloroformlisoamylalkohol 49:1

Die Zellen wurden vor der RNA-Isolierung in frischem Medium über Nacht kultiviert. Adhärent wachsende Zellen wurden mit 0,5 mM EDTA für 5 min bei 37°C abgelöst, mit 225g zentrifugiert und 2x in eiskaltem PBS gewaschen. Suspensionskulturen wurden ebenfalls bei 225g für 10 min zentrifugiert und 2x in eiskaltem PBS gewaschen. 1x10⁷ Zellen wurden in 1 ml Denaturierungslösung homogenisiert. Das Homogenat wurde mit 100 µl 2 M Na-Acetat, pH 4,0, versetzt und durch Inversion durchmischt. Anschließend wurde 1 ml wassergesättigtes Phenol zugegeben und 5 min bei Raumtemperatur inkubiert, dann wurde der Ansatz mit 200µl Chloroform/Isoamylalkohol versetzt und für 15 min bei 0°C-4°C inkubiert. Die wässrige Phase wurde von der organischen durch 20 min Zentrifugieren mit 15000 rpm bei 4°C getrennt. Die wässrige Phase wurde überführt und mit 1 Vol Isopropanol mindestens für 30 min bei -20°C gefällt. Die RNA wurde für 30 min mit 15000 rpm bei 4°C präzipitiert und in 300 µl Denaturierungslösung resuspendiert. Die RNA wurde wie oben angegeben gefällt, in 70% Ethanol gewaschen, das Pellet getrocknet, in entsprechendem Volumen H₂O_{DEPC} aufgenommen und bei -70°C gelagert.

Die Isolierung der mRNA erfolgte mit folgenden Puffern:

| | |
|---|---|
| 2x Bindungspuffer. | 20 mM Tris-HCl, pH 7,5 |
| | 1000 mM NaCl |
| | 2 mM EDTA |
| | 1% SDS |
| Waschpuffer: | OW2: 10 mM Tris-HCl |
| | 150 mM NaCl |
| | 1 mM EDTA |
| Elutionspuffer: | 5 mM Tris-HCl, pH 7,5, auf |
| | 70°C erhitzt. |

Die Isolierung der mRNA erfolgte mit Oligotex™-Partikeln von Qiagen im Batch-Verfahren. Das Protokoll wurde zur vollständigen Reinigung der mRNA wie folgt modifiziert: Die Gesamt-RNA wurde mit entsprechendem Volumen 2x Bindungspuffer versetzt und auf 65°C erhitzt. Der Ansatz wurde entsprechend der eingesetzten RNA-Menge mit 10% Oligotex™-Suspension nach Herstellerangaben versetzt (85 µl 10% Oligotex™ auf 1 mg Gesamt-RNA) und für 5 min bei 65°C unter Schütteln inkubiert. Anschließend wurde der Ansatz langsam auf Raumtemperatur abgekühlt und für weitere 10 min bei Raumtemperatur inkubiert. Die Oligotex™-Partikel wurden für 1 min bei 15000 rpm zentrifugiert, 2x mit 1 ml 1x Bindungspuffer und 2x mit 1 ml OW2 Waschpuffer gewaschen. Anschließend wurde die RNA mit 100 µl Elutionspuffer für 3 min bei 70°C eluiert. Die ribosomale RNA konnte in den meisten Fällen so nicht komplett entfernt werden, so daß die mRNA erneut an den gleichen Oligotex™-Partikeln aufgereinigt worden ist, indem das Volumen der eluierten mRNA auf 500 µl eingestellt worden ist und mit entsprechendem Volumen an 2x Bindungspuffer versetzt wurde. Die RNA wurde erneut bei 65°C denaturiert und anschließend für 10 min bei Raumtemperatur an die Oligotex™-Partikel hybridisiert. Die Partikel wurden wie oben gewaschen und anschließend wurde die mRNA mit 2x 25 µl Elutionspuffer für 3 min bei 70°C eluiert. Die mRNA wurde dann bei -70°C gelagert.

Plasmid DNA wurde entweder nach der alkalischen-Lyse Methode von Sambrook et al., A laboratory manual, 2nd. edition (1989) oder mit Qiaprep Spin Columns (Qiagen) nach Herstellerangaben aufgereinigt. Plasmide für die Sequenzierung wurden mit Qiawell8 Anionenaustauscher Säulen nach Herstellerangaben (Qiagen) aufgereinigt. Die Aufreinigung präparativer Plasmid DNA erfolgte aus 100-200 ml LB-Kulturen mit entsprechendem Antibiotikum mit Qiatip500-Säulen (Qiagen).

Die analytischen Restriktionsansätzen der DNA wurden mit 0,5-1 µg Plasmid-DNA und 2,5 U der jeweiligen Restriktionsenzyme mit dem vom Hersteller mitgelieferten 10x Puffer in einem Volumen von 10-20 µl durchgeführt. Die enstandenen DNA-Fragmente wurden in der Agarosegelelekrophorese (SeaKem^{®} LM, FMC) analysiert. Als Längenstandard wurde die 1 kb Leiter (BRL Gibco) standardmäßig in die Agarosegelelektrophorese eingesetzt.

In präparativen Restriktionsansätzen wurden bis zu 5 µg Plasmid-DNA in einem 100 µl Ansatz mit den entsprechenden Restriktionsenzymen inkubiert, wobei Doppelansätze entsprechend den Herstellerangaben mit dem optimalen Puffer versetzt worden sind. Die DNA-Fragemente wurden nach wie bereits oben beschrieben isoliert.

Gelelution von DNA-Fragmenten erfolgte nach Herstellerangaben mit Qiaex II (Qiagen) aus Agarose. Für die Subklonierung der DNA-Fagmente wurden diese in 1 % Low Melting Agarose (Nusieve^{®} GTG, FMC) aufgetrennt, ausgeschnitten und die Agarose bei 67°C für 10 min geschmolzen. Die geschmolzene Agarose wurde mit 4 bis 5 Volumen TE-Puffer (67°C) versetzt, so daß der Agaroseanteil unter 0,2% gesunken ist. Anschließend wurde 2x mit 1 Vol TE gesättigtes Phenol, 1 x 1 Vol Phenol/Chloroform 25:1 und 1 x 1 Vol Chloroform/Isoamylalkohol 49:1 die DNA extrahiert. Die DNA wurde mit 0,3 M Na-Acetat und 2 Vol 100% Ethanol für 20 min bei -20°C gefällt und anschließend durch Zentrifugation für 15 min bei 4°C präzipitiert. Nachdem das DNA-Pellet in 70% Ethanol gewaschen und getrocknet worden ist, wurde es in entsprechendem Volumen 10 mM Tris, pH 8,5 resuspendiert.

Zur Isolierung der ET spezifischen Antigene wurde eine cDNA Expressionsbibliothek von der ET Zelllinie VH-64 im λ ZAP Express™ (Stratagene) nach entsprechendem Protokoll des Herstellers erstellt.

Dazu wurde die mRNA nach den bereits oben erläuterten Methoden aus der ET-Zelllinie VH-64 isoliert. In die cDNA Synthese wurden 10 µg mRNA eingesetzt, dabei wurden folgende Modifikationen abweichend vom Firmenprotokoll durchgeführt: Die mRNA wurde für die Erststrangsynthese mit folgenden Agenzien versetzt, nachdem sie für 5 min bei 70°C denaturierte:

| | |
|---|---|
| 5,0 µl | 10x Erststrang Synthese Puffer |
| 3,0 µl | Nukleotide Mixture mit 5-Methylcytosine |
| 2,0 µl | Linker-Primer (2,8µg) |
| 20,0 µl | mRNA (0,5µg/µl) |
| 17,5 µl | H₂O_{DEPC} |
| 1,0 µl | RNase Inhibitor (40U) |
| 48,5 µl | Ansatz |

Der Ansatz wurde 10 min bei Raumtemperatur für das Primerannealing inkubiert, dabei handelt es sich um Poly (dT) Primer, der am 5'-Ende durch die Erkennungssequenz des *Xho* I-Restriktionsenzyms flankiert wird. Anschließend wurde der Ansatz mit 1,5 µl MMLV-Reverse Transkriptase (75 U) versetzt. Die cDNA-Erststrangsynthese wurde kontrolliert, indem 5 µl des Ansatzes zu 0,5 µl [α-³²P]dATP (800Ci/mmol) gegeben wurden. Beide Ansätze wurden 1 h bei 42°C inkubiert.

Die Doppelstrangsynthese wurde in folgendem Ansatz für 2½ h bei 16°C durchgeführt:

| | |
|---|---|
| 45,0 µl | cDNA-Erststrang Ansatz |
| 20,0 µl | 10x Zweistrang-Puffer |
| 6,0 µl | Zweitstrang-Nukleotide |
| 113,9 µl | H₂O_{DEPC} |
| 2,0 µl | [α³²-P] dNTP |
| 2,0 µl | RNase H (3U) |
| 11,1 µl | DNA Polymerase I (100 U) |
| 200,0 µl | Ansatz |

Die überstehenden Enden der cDNA nach der 2. Strangsynthese wurden mit 5 U Pfu-DNA Polymerase für 30 min bei 72°C aufgefüllt (23 µl blunting dNTP-Mix, 2,0 µl *Pfu).* Die Erst- und Zweitstrangsynthese wurden in der denaturierenden Agarosegelelektrophorese (300 mM NaOH, 150 mM NaCl, 1 % Agarose) kontrolliert. Die cDNA wurde Phenol/Chloroform extrahiert, mit 0,3 M Na-Acetat und 2 Vol. 100% Ethanol über Nacht bei -20°C präzipitiert und für 1 h mit 15000 rpm bei 4°C zentrifugiert. Die präzipitierte cDNA wurde mit 70% Ethanol gewaschen und anschließend luftgetrocknet. Die cDNA wurde in 8 µl Eco RI (0,4µg/µl) Adaptern aufgenommen.

Für die gerichtete Klonierung in den λ ZAP-Express™ wurde die cDNA/Eco RI Adapter (8 µl) mit 1 µl 10x Ligasepuffer und 1 µl Ligase versetzt und für 2 d bei 4°C ligiert. Die Ligationsreaktion wurde durch die Inkubation bei 70°C für 30 min gestoppt. Die Ligation der Adapter wurde im PAGE/TBE Gel kontrolliert, indem in einem Kontrollreaktionsansatz die cDNA mit [α-³²P]ATP markierten Eco RI-Adaptern ligiert wurde. Dieser Ligationsansatz wurde gegenüber der cDNA allein und der cDNA mit markierten Adaptern ohne Ligase im PAA Gel aufgetrennt.
Die Adapter wurden anschließend in folgendem Ansatz für 30 min bei 37°C phosphoryliert:

| | |
|---|---|
| 10,0 µlµ | cDNA mit ligierten Eco RI Adaptern |
| 1,0 µl | 10x Ligase Puffer |
| 2,0 µl | 10 mM rATP |
| 6,0 µl | H₂O |
| 1,0 µl | T4 Polynukleotide Kinase (10U) |
| 20,0 µl | Ansatz |

Die Kinase wurde bei 70°C für 30 min inaktiviert und anschließend die cDNA mit ligierten Adaptern mit *Xho* I für 1,5 h bei 37°C inkubiert.

| | |
|---|---|
| 20,0 µl | cDNA mit phosphorylierten Adaptern |
| 28,0 µl | *Xho* I Puffer |
| 3,0 µl | *Xho* I (120 U) |
| 51,0 µl | Ansatz |

Die cDNA wurde anschließend ihrer Größe entsprechend über eine C4-LB Sepharose Säule (Pharmacia) fraktioniert. Dazu wurde die cDNA mit 5 µl 10x STE (100 mM NaCl; 10 mM Tris/HCl, pH8,0; 1 mM EDTA, pH8,0), 3µl 2% Bromphenolblau und 15 µl 80% Glycerin versetzt. Es wurden 40 Fraktionen mit einem Volumen von ∼100 µl entnommen. Die eingebaute Radioaktivität der einzelnen Fraktionen wurde im Szintillator (Beckmann) gemessen. Die Länge der cDNA wurde im 1% TBE-Agarosegel kontrolliert, indem 1/20 der Fraktionen aufgetrennt wurde.

Die Fraktionen, die cDNA Moleküle größer als 500 bp enthielten, wurden vereinigt und mit 1 Vol 100% Ethanol gefällt. Die mit 70% Ethanol gewaschene cDNA wurde für die Vektor-Ligation in 3 µl H₂O aufgenommen und in folgendem Ansatz für 2 Tage bei 4°C in den λ ZAP Express™ ligiert:

| | |
|---|---|
| 3,0 µl | größenfraktionierte cDNA mit phosphorylierten Adaptern |
| 0,5 µl | 10x Ligasepuffer |
| 0,5 µl | 10 mM rATP |
| 1,0 µl | λ ZAP-Express™ Arme (1 µg) |
| 0,5 µl | T4 DNA-Ligase |
| 5,5 µl | Ansatz |

Die Verpackung der in den λ Zap Express™ ligierten cDNA wurde nach den Instruktionen des Gigapack II Gold (Stratagene) durchgeführt.

Zur Amplifikation der cDNA Bibliothek wurde eine Kolonie des *E*. *coli* Stammes XL1-blue MRF' über Nacht bei 30°C mit 200 rpm oder 4-6 h bei 37°C bis zu einer OD₆₀₀ von 0,5 in LB-Medium mit 12,5 µg/ml tetracyclin-Sulfat angezogen. Die Bakterien wurden mit 2000 rpm für 10 min pelletiert und entsprechend in 10 mM MgSO₄ Lösung aufgenommen. 600 µl XL1-blue MRF' wurden mit dem entsprechenden Volumen an Primärphagen versetzt, und für 15 min bei 37°C zur Adhäsion der Phagen an die Bakterien inkubiert. Anschließend wurden 6,5 ml NZY Top-Agarose (48°C) zugegeben und auf eine 150 mm² NZY-Agarplatte ausplattiert, Die Agarplaten wurden für 6-8 h bei 37°C inkubiert, so daß die Phagenplaques nicht größer als 1-2 mm wurden. Die Platten wurden mit 10 ml SM-Puffer überschichtet und bei 4°C über Nacht gelagert. Anschließend wurden diese noch 2 Stunden bei Raumtemperatur unter leichtem schütteln inkubiert. Der SM-Puffer wurde abgenommen, die 150 mm Agarplatten mit 2 ml SM-Puffer gewaschen und anschließend mit 5% Chloroform versetzt, 15 min bei Raumtemperatur inkubiert und Zelltrümmer durch Zentrifugieren für 10 min mit 5000 g entfernt.

Um die *in vivo* Excision des pBK-CMV ducrhzuführen, wurde eine Kolonie vom XL1-blue MRF' über Nacht in LB/0,2% Maltose/10 mM MgSO₄ Medium und vom XLOLR in LB-Medium bei 30°C angezogen. Die Bakterien wurden mit 1000 g für 10 min zentrifugiert und in 10 mM MgSO₄ mit der OD₆₀₀ von 1,0 aufgenommen. Die isolierten λ-Phagenklone wurden in 500 µl SM-Puffer mit 20 µl Chloroform aufgenommen und über Nacht bei 4°C gelagert. Zur *in vivo Excision* der X-Phagenklone wurden 200 µl vom XL1-blue MRF' mit 250 µl vom λ-Phagenstock und 1 µl *ExAssist*™ (>1x10⁶ pfu/µl) versetzt und für 15 min bei 37°C inkubiert. Der Ansatz wurde mit 3 ml LB-Medium versetzt und für 2½-3 h bei 37°C mit 225 rpm inkubiert. Die Bakterien wurden für 20 min bei 70°C hitzeinaktiviert und mit 1000 g für 15 min abzentrifugiert. Der Überstand, der die pBK-CMV Phagemide als einzelsträngige filamentös Phagen enthält, überführt. Um die Phagemide in doppelsträngige Plasmide zu überführen, wurden 10-100 µl des Phagemidüberstandes mit 200 µl der XLOLR-Suspension versetzt und für 15 min bei 37°C inkubiert. Der Ansatz wurde mit 300 µl LB-Medium versetzt und für 45 min bei 37°C inkubiert. 200 µl des Ansatzes wurden auf LB-Agarplatten mit 50 µg/ml Kanamycin ausplattiert und über Nacht bei 37°C inkubiert. Die *in vivo Excision* überführt den λ ZAP Express™ in das Phagemid pBK-CMV.

Für den Immunoscreen der λ ZAP Express™ cDNA Expressionsbibliothek wurden folgende Ansätze verwendet:

| | |
|---|---|
| TBS | 10 mM Tris/HCl, pH 7,5 |
| | 150 mM NaCl |
| Blockierungspuffer | TBS1% BSA |
| | 0,05 Tween 20 |
| TBST | TBS |
| | 0,1% Tween 20 |
| AP-Puffer | 100 mM Tris/HCl, pH 9,5 |
| | 100 mM NaCl |
| | 2 mM MgCl₂ |
| AP-Substratpuffer | 6,6 µl 50 mg/ml NBT in 70% DMF |
| | 3,3 µl 50 mg/ml BCIP in 100% DMF pro ml AP-Puffer |

Eine Einzelkolonie vom XL1-blue MRF' wurde in LB/0,2% Maltose/10 mM MgSO₄ Medium bis zu einer optischen Dichte von OD₆₀₀ 0,5 angezogen. Die Bakterien wurden mit 2000 rpm für 10 min bei 4°C zentrifugiert und im entsprechenden Volumen 10 mM MgSO₄ aufgenommen. Der Primärscreen der Expressionsbibliothek wurde mit 5x10⁵ pfu durchgeführt, das entspricht dem Umfang der Primärexpressionsbibliothek. Dazu wurden 600 µl der XL1-blue MRF' Suspension mit 5x10⁴ pfu pro 150 mm² Petrischale (Greiner) versetzt und 15 min bei 37°C inkubiert. Die Ansätze wurden mit 7 ml NZY-Top-Agarose (48°C) versetzt und auf NZY-Agarplatten ausplattiert. Die Agarplatten wurden für 3,5 h bei 42°C inkubiert, anschließend mit Nitrozellulosemembranen (HATF, Millipore), die vorher in 10 mM IPTG geschwenkt und getrocknet wurde, bedeckt und für 4,5 h bei 37°C inkubiert. Die markierten Membranen wurden entfernt, 30 min in TBST gewaschen und bei 4°C gelagert. Es wurde ein zweiter Satz Membranen von den Agarplatten gezogen, indem diese bei 37°C über Nacht inkubiert und anschließend ebenfalls in TBST gewaschen wurde.

Die Filter wurden in 1 ml/cm² Blockierungspuffer für mindestens 30 min bei Raumtemperatur inkubiert. Die Membranen des Doppelscreens wurden gemeinsam mit den Antikörpern im Blockierungspuffer versetzt, dabei wurden die Hybridomaüberstande der ET spezifischen monoklonalen Antikörper zu gleichen Teilen vereinigt und in einem Verhältnis von 1:100 zum Blockierungspuffer gegeben. Die Inkubation mit den Antikörpern wurde über Nacht bei 4°C auf einem Horizontalschüttler (5 rpm) durchgeführt. Anschließend wurden die Membranen für 3x 10 min in TBST einzeln gewaschen und mit dem 2. Antikörper Ziege-α-Maus AP konjugiert (1:1000, DAKO) in Blockierungspuffer über Nacht bei 4°C inkubiert. Die Membranen wurden wie oben gewaschen und in AP-Puffer äquilibriert. Die Membranen wurden mit AP-Substratpuffer versetzt und für 3 h entwickelt. Die potentiellpositiven Klone wurden ausgestochen und in 500 µl SM-Puffer mit 20 µl Chloroform bei 4°C gelagert. Anschließend wurden die Phagenklone durch Rescreens aufgereinigt und durch die *in vivo Excision* in die Plasmidform überführt.

Im Immunoscreen von transformierten E. coli Kolonien fand folgender Lysepuffer Verwendung:

| | |
|---|---|
| Lysepuffer: | 100 mM Tris/HCl, pH 7,8 |
| | 150 mM NaCl |
| | 5 mM MgCl₂ |
| | 1 mg/ml Pankreas DNase I |
| | 40 mg/ml Lysozym |

Die transformierten Bakterien wurden auf LB-Agarplatten ausgestrichen, bei 37°C über Nacht angezogen und anschließend in invertierter Stellung bei 4°C gelagert. Die Agarplatte wurde dann mit in 10 mM IPTG geschwenkten Nitrocellulose Filter überlagert und für 2-4 h bei 37°C inkubiert. Nach Entfernen der Filter wurden diese zum Abtöten der Bakterien 15 min Chlordämpfen ausgesetzt. Die Nitrocellulose-Membranen wurden dann für 12-16 h mit -1 ml/cm² Lysepuffer inkubiert und anschließend 2x 30 min in TBST bei Raumtemperatur gewaschen. Die Antikörpermarkierung wurde dann wie oben beschrieben durchgeführt. Der Hybridomaüberstand des 1. Antikörpers wurde 1:50 in Blockierungspuffer eingesetzt. Die Detektion erfolgte ebenfalls wie bereits beschrieben.

Für die Transformation von *E. coli* wurde verwendet:

| | |
|---|---|
| TSS: | 10% PEG 8000 |
| | 5% DMSO |
| | 50 mM MgSO4 |
| | in LB-Medium, pH 6,5 |

Die Transformation von *E*. *coli* erfolgte nach der Methode von Chung et al., Proceeding of the National Academy of Science 86: 2172-2175 (1989). Dabei wurden die jeweiligen Bakterienstämme aus einer Übemachtkultur 1:100 in LB-Medium ohne Antibiotikum überimpft und bis zur frühen logarithmischen Wachstumsphase (OD₆₀₀ 0,3-0,4) kultiviert. Die Zellen wurden mit 1000g für 10 min bei 4°C geerntet, in 1/10 des Ausgangsvolumens in TSS aufgenommen und zu 100 µl Aliquots im Ethanol/Trockeneisbad schockgefroren. Die Zellen wurden direkt in die Transformation eingesetzt oder bei -70°C gelagert.

Zur Transformation wurden die Zellen auf Eis aufgetaut, mit 0,1-1 µg Plasmid-DNA versetzt und für 30 min auf Eis inkubiert. Nach einem 1 minütigen Hitzeschock von 42°C wurde der Ansatz mit 0,9 ml LB/20 mM Glucose versetzt und für 1 h bei 37°C mit 225 rpm inkubiert. Die transformierten Zellen wurden mit 1000g für 10 min geemtet und mit 100 µl LB-Medium auf LB-Agarplatten mit entsprechendem Antibiotikum ausplattiert.

Eukaryotische Zellen wurden nach dem Protokoll von Boehringer Mannheim mit FuGENE6™ transient transformiert. Dazu wurden die COS-7 Zellen am Tag vor der Transfektion mit 2-3x10⁵ Zellen pro cm² in MEMα-Medium/10% FCS in 6-Well Kulturschalen ausgesät. Am folgenden Tag wurden 4 µg FuGENE6™ direkt in 100 µl MEMα aufgenommen und 5 min bei Raumtemperatur inkubiert. Zwei Mikrogramm der jeweiligen Plasmide, die aus einer Maxi-Präparation über Qiagen-Säulen aufgereinigt worden sind, wurden in 1,5 ml Eppendorfgefäße vorgelegt und anschließend das FuGENE6™/MEMα-Gemisch tropfenweise zur DNA gegeben. Der Ansatz wurde 15 min bei Raumtemperatur inkubiert und dann tropfenweise zu den Zellen, die mit frischem MEMα-komplett Medium versetzt worden sind, gegeben. Die Zellen wurden für 24 h, 48 h und 72 h ohne Mediumswechsel inkubiert und anschließend mit 0,5 mM EDTA für 5-10 min bei 37°C abgelöst. Zur durchflußzytometrischen Analyse wurden die Zellen wie bereits oben beschrieben behandelt.

Zur Bestimmung der Transfektionseffizienz wurde der λ ZAP-Express™ mit intaktem β-Galactosidase Gen aus der VH-64 cDNA Expressionsbibliothek isoliert, in die Phagemid Form überführt und anschließend die *Multicloning site* sequenziert.

Als Reportergen wurde das humanisierte *GFP* aus dem Vektor pEGFP-1 (Clontech) verwendet. Das pEGFP-1 wurde aus dem *dam* negativen *E.coli-*Stamm GM-84 isoliert und GFP mit den Restriktionsenzymen *Bam* HI und *Xba* I ausgeschnitten. Nach einer Gelaufreinigung wurde dieses dann gerichtet in den pBK-CMV kloniert.

Die Deletionsklone wurden mit dem Exo III/Mung Bean Nuklease Kit von Stratagene hergestellt. Dazu wurden die zu deletierenden Klone mit einem 5' bzw. "Blunt End" schneidenden und einem 3'-Überhang produzierenden Restriktionsenzym behandelt, welches das Vektor-Rückgrat vor Exonuklease III schützt. Das pBK-CMV Rückgrat wurde für die 5' Deletionsklone durch Inkubation mit *Bss* HII und anschließendem Auffüllen der überhängenden Enden mit α-thio Phosphat dNTPs geschützt. Zur Herstellung der 3'-Deletionsklone wurde das pBK-CMV Rückgrat durch das Öffnen der Phagemidklone mit den 3'-Überhängen produzierenden Restriktionsenzymen *Kpn* I bzw. *Bst* XI geschützt. Die cDNA wurde für die Exonuklease III zugänglich gemacht, indem sie für die 5' Deletionen mit *Bam* HI und für die 3' Deletionen mit *Xba* I inkubiert worden sind. Nach einer Phenol/Chloroform und Chloroform/Isoamylalkohol 49:1 Extraktion wurden jeweils 5 µg geöffnetes Phagemid pro Minute Exonuklease III Inkubation eingesetzt. Die Exonuklease III wurde mit 20 U/pmol 5'-Ende eingesetzt und der Ansatz bei 30°C inkubiert. Die entnommenen Aliquots wurden in Stoppuffer (20 µl 10x Mung Bean Puffer auf 155 µl H₂O) überführt und die Exonuklease III bei 68°C für 15 min deaktiviert. Die einzelsträngigen 5'-Überhänge wurden mit 15 U Mung Bean Nuklease bei 30°C für 30 min entfernt. Die Ansätze wurden mit Phenol/Chloroform extrahiert und mit 0,3 M Na-Acetat und 2 Vol Ethanol gefällt. Das Pellet wurde mit 70% Ethanol gewaschen, getrocknet und in 15 µl TE-Puffer aufgenommen. Die Exonuklease III und Mung Bean Nuklease behandelten Klone wurden mittels der T4 DNA Ligase nach Herstellerangaben religiert und in XL1-blue MRF' transformiert. Die Klone wurden auf Masterplatten übertragen und nach 8 h Inkubation bei 37°C mit der Minipräparationsmethode, die von Good and Nazar, Biotechniques 22: 404-406 (1997) nach Klonen mit der richtigen Größe durchsucht.

In der DNA-Sequenzierung wurden folgende Ansätze verwendet:

| | |
|---|---|
| 10xTBE (Sequenzierung): | 1 M Tris/HCl, pH8,9 |
| | 89 mM Borsäure |
| | 1 mM EDTA |
| Sequenzgel: | 5% Longrangermatrix™ |
| | 7 M Harnstoff |
| | 1,5x TBE |
| | 0,5% APS |
| | 0,05% TEMED |
| Laufpuffer: | 0,5x TBE |

Die Sequenzierungen wurden mit dem automatischen Laser Fluoreszenz Sequenzierer ALF*express* (Pharmacia) durchgeführt. Die Sequenzierung der Plasmid-DNA wurde nach der Kettenabbruchmethode von Sanger et al., Proceedings of the National Academy of Science 80: 3963-3965 (1977) entweder mit der T7-DNA Polymerase oder durch Cyclesequenzierung mit der Thermosequenase™ (Amersham) durchgeführt.

Fünf µg der doppelsträngigen Plasmid-DNA, die über Anionenaustauscher-Säulen Qiawell8 (Qiagen) isoliert wurden, wurden für die T7-Reaktion nach der Quick-Denaturierungs Methode nach Zimmermann et al., Nucleid. Acids.Res. 18: 1067 (1990) behandelt, und anschließend die Sequenzreaktion mit dem T7 Autoread Kit (Pharmacia) nach Herstellerangaben durchgeführt.

Die Cyclesequenzierung wurde mit 1 µg Plasmid-DNA nach Herstellerangaben mit Primer (1 pmol) und den Thermosequenase/ddNTP-Reagentien durchgeführt. Die Cyclebedingungen wurden in einen 3-Schritt Cyclus geändert, mit einer initiale Denaturierung von 5 min bei 95°C gestartet und anschließend 30 Zyklen mit der Denaturierung für 30 sec bei 95°C, dem Annealing für 15 sec bei der entsprechenden Primer-Schmelztemperatur (Tm) und einer Elongation von 30 sec bei 72°C durchgeführt. Die Cyclesequenzierung wurde mit einer 5 min Elongationsphase abgeschlossen. Die Reaktion wurde mit 1/3 Vol Stopp-Lösung (5 mg/ml Dextrin in deionisiertem Formamid) versetzt und anschließend bei 85°C für 3 min denaturiert. Die Primer für beide Sequenzierungsmethoden waren am 5'-Ende mit dem Chromophor Cy-5 markiert.

| **Primer** | **Sequenz 5' → 3'** | **Tm [°C]** | **Bp** |
|---|---|---|---|
| T7 (-) | GTAATACGACTCACTATAGGGC | 54,9 | 22 |
| T3 (+) | AATTAACCCTCACTAAAGGG | 55,1 | 20 |
| M13-universal | CGACGTTGTAAAACGACGGCCAGT | 72,7 | 24 |
| M13-reversal | CAGGAAACAGCTATGAC | 47,8 | 17 |

Die Sequenzierungsreaktion wurde in einem 0,5 mm Gel mit folgender Matrix getrennt:
5% Longrangergel™
7 M Harnstoff
1,5x TBE
0,5% APS
0,05% TEMED.

Das Sequenzgel lief mit 0,5x TBE Laufpuffer bei konstanten 25 Watt und 51°C für 900 min. Die Sequenzen wurden mit der ALFwin 1.1 Software prozessiert und dem Auswerteprogramm DNASIS (Hitachi) analysiert. Die zusammengesetzten Sequenzen wurden mit den Alignmentprogrammen BLAST vom NCBI (Web Seite: http://www.ncbi.nlm.nih.gov/) und FastA vom EBI (Web Seite: http://www.ebi.ac.uk_ebi_home.html) auf homologe Sequenzen analysiert.

Der Nachweis spezifischer DNA- und RNA-Sequenzen erfolgte mit folgenden Lösungen:

| | |
|---|---|
| 20x SSC: | 3 M NaCl |
| | 300 mM Na-Citrat, pH 7,0 |
| Denaturierungslösung | 0,5 M NaOH |
| | 0,5 M NaCl |
| Neutralisierungslösung | 0,5 M Tris/HCl, pH 8,0 |
| | 1,5 M NaCl |
| | 10x MOPS |
| | 200 mM MOPS |
| | 50 mM NaAc |
| | 10 mM EDTA, pH 7,0 |
| RNA-loading-Dye | 20% Ficoll |
| | 20 mM EDTA |
| | 0,15% Bromphenol Blau |
| RNA-Präybridisierungslösung | 50% deionisiertes Formamid |
| | 6x SSC |
| | 5x Denhardt's Reagent |
| | 0,5% SDS |
| | 100 µg/ml Heringssperma |
| RNA-Hybridisierungslösung | 50% deionisiertes Formamid |
| | 6x SSC |
| | 0,5% SDS |
| | 100 µg/ml Heringssperma |

Die radioaktive Markierung von DNA-Fragmenten erfolgte nach der "random priming" Methode mit dem NEBlot Kit (NEB) nach den Anweisungen des Herstellers, die auf der "random-priming" Methode nach Feinberg und Vogelstein, Analytical Biochemsitry 178: 6-13 (1983) beruht. Die radioaktive Markierung von *in vitro* Transkripten wurde mit dem T7/T3 Transkription Kit (Stratagene) durchgeführt. Die Sonden wurden durch Fällen mit 0,3 M Na-Acetat, pH5,2, und 2 Vol eiskaltem EtOH gereinigt, mit 2 Vol 70% EtOH gewaschen, getrocknet und in entsprechendem Volumen H₂O oder TE-Puffer aufgenommen, oder in Zentrifugensäulchen Microcon-100 (Millipore) gereinigt.

Die nicht-radioaktive Markierung wurde mit dem Direkt Labeling Kit von Amersham durchgeführt.

Für den Southem Blot wurde die genomische DNA entsprechend den Angaben der Hersteller mit verschiedenen Restriktionsenzymen inkubiert und 3-5 µg der geschnittenen DNA wurden in einem 0,7% TBE-Agarosegel getrennt. Anschließend wurde das Gel für 15-30 min in 10 Gelvolumen 0,25 M HCl inkubiert. Die genomische DNA wurde dann 2x 15 min mit 10 Vol Denaturierungslösung denaturiert und 2x 10 min mit Neutralisierungslösung behandelt. Die DNA wurde im Kapillartransferverfahren mit 20xSSC auf Hybond N+ (Nycomed-Amersham) transferriert. Anschließend wurde der Blot für 30 min bei 120°C fixiert. Der Größenstandard wurde vom Blot abgetrennt, mit 0,3% Methylenblau in 0,3 M Na-Acetat Lösung für 2 min angefärbt und anschließend mit Wasser für 5 min bei Raumtemperatur entfärbt. Der fixierte Southem Blot wurde im Hybridisierungspuffer (Amersham) 15 min bei 55°C prähybridisiert. Anschließend wurde die markierte Sonde zum Hybridisierungspuffer gegeben und diese über Nacht bei 55°C im Hybridisierungsofen (Biometra) hybridisiert. Nach der Hybridisierung wurde der Blot 2x15 min bei 60°C in 1. Waschpuffer und 2x5 min im 2. Waschpuffer gewaschen. Die Blots wurden mit CPD^{Star} versetzt und 5 min bei Raumtemperatur inkubiert. Das überschüssige CPD^{Star} wurde entfernt und der Blot eingeschweist. Die hybridisierte Sonde wurde auf dem Blot durch Belichtung eines Hyper-ECL Films (Amersham) für einige Minuten bis zu mehreren Stunden nachgewiesen.

Für die Proteinanalysen in der SDS-PAGE fanden folgende Materialien Verwendung:

| | |
|---|---|
| Acrylamid-Stammlösung: | 30% Acrylamid |
| | 0,75% Bis-Acrylamid (Biozym) |
| | 4x (1,5 M) Tris/HCl, pH 8,8 |
| | 4x (0,5 M) Tris/HCl, pH 6,8 |
| | 10% Ammoniumpersulfat (APS) |
| | N, N, N', N'-tetramethylethylenediamine (TEMED) |
| 5% Acrylamid-Sammelgel: | 125 mM Tris/HCl, pH 6,8 |
| | 0,1% SDS |
| | 0,09% TEMED |
| | 0,09% APS |
| Elektrophoresepuffer: | 20 mM Tris 190 mM Glycin |
| | 0,1% SDS |
| 2x Probenpuffer: | 125 mM Tris/HCl, pH 6,8 |
| | 20% (v/v)Glycerin |
| | 2% (v/v) b-Mercaptoethanol |
| | 1% (w/v) Bromphenolblau |
| | 4% SDS |

Die Acrylamidkonzentration für das Trenngel wurde entsprechend der gewünschten Gelkonzentration hergestellt, TEMED und APS mit einer Endkonzentration von 0,02% zugegeben und damit die vorbereiteten Gelkammem beschickt.

Die sedimentierten Zellinien (1,25x10⁶ Zellen) bzw. transformierten *E.coli* Zellen (25 µl einer Übernachtkultur) wurden in 1 x Probenpuffer aufgenommen, diese Proben wurden für 5 min bei 95°C denaturiert und anschießend auf das Gel aufgetragen.

Im Western Blot wurden folgende Lösungen verwendet:

| | |
|---|---|
| Transfer-Puffer: | 25 mM Tris/HCl |
| | 192 mM Glycin, pH 8,3 |
| | 20% (v/v) Methanol |
| Coomassie-Gelfärbungslösung | 0,05% (w/v) Coomassie |
| | Brilliant Blue R-250 |
| | 50% (v/v) Methanol |
| | 10% (v/v) Essigsäure |
| Gel-Entfärber | 10% (v/v) Essigsäure |
| | 20% (v/v) Methanol |
| Transferpuffer | 25 mM Tris/HCl |
| | 192 mM Glycin, pH 8,3 |
| | 20% (v/v) Methanol |
| Membran Färbelösung | 0,5% (w/v) Ponceau S (Sigma) |
| | 1% (v/v) Essigsäure |
| Membran Entfärber | 5% (v/v) Essigsäure |

Die Proteine wurden mit einer Spannung von 20 V und 300 mA für 2 h von dem Gel auf die Membran geblottet.

Zur Detektion der Antigene durch den Antikörper wurde die Membran für 3 h bei Raumtemperatur in Blockierungspuffer inkubiert und anschließend mit frischem Blockierungspuffer, der mit dem Hybridomaüberstand eines ET spezifischen Antikörpers im Verhältniss von 1:250 versetzt wurden, über Nacht bei 4°C inkubiert. Anschließend wurde die Membran 2x 15 min mit TBST (2.2.4.5) gewaschen und mit dem 2. Antikörper Kaninchen-α-Maus-Ig (H+L) AP-konjugiert (DAKO) 1:1500 in Blockierungspuffer für 2 h bei Raumtemperatur inkubiert. Die Membran wurde wie vorher beschrieben gewaschen und mit AP-Substratpuffer entwickelt.

### SEQUENZPROTOKOLL

ALLGEMEINE ANGABEN:
   ANMELDER:
      NAME: E. Marion Schneider
      STRASSE: Sebastian-Kneipp-Weg 25
      ORT: Ulm
      LAND: Deutschland
      POSTLEITZAHL: 89075
   BEZEICHNUNG DER ERFINDUNG: Ewing Tumor Antigen und Nukleinsäure
   ANZAHL DER SEQUENZEN: 1
   COMPUTERLESBARE FASSUNG:
      DATENTRÄGER: Diskette
      COMPUTER: IBM PC-kompatibel
      BETRIEBSSYSTEM: Windows 95
      SOFTWARE: PatentIn 2.1
   DATEN DER JETZIGEN ANMELDUNG:
      ANMELDENUMMER: 10212779
ANGABEN ZUR SEQ ID NO: 01
   SEQUENZCHARAKTERISTIKA:
      LÄNGE: 3457 bp
      ART:Nukleinsäure
      STRANGFORM: Einzelstrang
      TOPOLOGIE: linear
   ART DES MOLEKÜLS: cDNA zu mRNA
   HYPOTHETISCH: nein
   ANTISENSE: nein
URSPRÜNGLICHE HERKUNFT:
   ORGANISMUS: Homo sapiens
   ZELLTYP: Ewing Tumor Zellinie
   ZELLINIE: VH-64
   UNMITTELBARE HERKUNFT:
      BIBLIOTHEK: λZAP Express^{™} Expressionsbibliothek der ET-Zellinie
         VH-64
      CLONE: 3.3.1; 6.1.1; 8.1.1; 9.2.2
   POSITION IM GENOM
      CHROMOSOM/SEGMENT: 2p13-15
   MERKMALE:
      CODIERENDE SEQUENZ: 403 bis 2925 bp
      1. POLY A-BEREICH: 3057 - 3062 bp
      2. POLY A-BEREICH: 3269 - 3274 bp
      3. POLY A-BEREICH: 3408 - 3413 bp
      5'UTR-BEREICH: 1 - 402 bp
      3'UTR-BEREICH: 2926 - 3480 bp
      ARE-BEREICH: 3051 - 3056 bp
      KOZAK SEQUENZ:400 - 406 bp
      ECO RI ADAPTER: 1 - 14 bp
      SMA I ERKENNUNGSSEQUENZ: 304 - 309 bp
      ECO RI ERKENNUNGSSEQUENZ: 474 - 479 bp
      HIN DIII ERKENNUNGSSEQUENZ: 1691 - 1696 bp
      SPE I ERKENNUNGSSEQUENZ:1846 - 1851 bp
      XHO I ERKENNUNGSSEQUENZ: 3475 - 3457 bp
      STARTCODON: 403 405 bp
      STOPPCODON: 2923 - 2925 bp
      1. ORF IN 5'-UTR-BEREICH: 166 - 258 bp
      2. ORF IN 5'-UTR-BEREICH: 221 - 292 bp
      3. ORF IN 5'-UTR-BEREICH: 147 - 356 bp
   ERMITTLUNGSMETHODE: alle Merkmale wurden mit dem
   Auswerteprogramm
      DNAsis von Hitachi ermittelt.
   SONSTIGE ANGABEN:
      GC-GEHALT DES 5'-UTR-BEREICHS: 62,18 %
      GC-GEHALT DES 3'-UTR-Bereichs: 26,92 %
      GC-GEHALT DES ORF: 26,98 %
   BILOGISCHE FUNKTIONEN DES TRANSKRIPTS:
      - das native ETAA16 (und/oder Teile daraus) übernimmt (übernehmen) regulative Funktionen in der Interaktion mit cytolytischen Zellen
      - das Polypeptid (oder Teile daraus) liefert (liefern) Epitope des monoklonalen Antikörper SN58/1857.15.30 gegen Zellen des Ewing Tumors
   SEQUENZBESCHREIBUNG: SEO ID NO: 1

gaattcggca cgagcttgcg cgcgccccac cgaccaaaat ggcggctgcc gttggtgcgg 60

ggtgcggttt gtagtgctgt tgccctactc atccctttgc aaaatgtgaa aagaagaaag
120

cggctggtgg aggcggccca taggcaatga gtcggcgaag gaaacatgat gacagcccta
180

gcccgaagaa aacgccgcac aaaacagtgg cggcggagga atgcggctcg gtggtcgagc
240

cagggaggag gcggctgaga tcggcccgcg gttcgtggcc ctgcggggct agagaggggg
300

cctcccgggc cagtgcggca gcgagagcag cctccgaccg ccgccctgtg cagtaaaagt
360

aaccccgagg aaaggtatga aacaccaaag agagcgctga aa atg gac tca ctg
414
                                                Met Asp Ser Leu
                                                 1

tca tct tcc ttc agt tct cct aat gat cca gat gga cag aat gat atc
462
Ser Ser Ser Phe Ser Ser Pro Asn Asp Pro Asp Gly Gln Asn Asp Ile
  5                   10                  15                  20

ttt tgg gat cag aat tct cca ttg aca aag cag tta ggt aaa gga aga
510
Phe Trp Asp Gln Asn Ser Pro Leu Thr Lys Gln Leu Gly Lys Gly Arg
                25                  30                  35

aaa aaa cag att tac acc aca gat agt gat gag att tca cat att gtt
558
Lys Lys Gln Ile Tyr Thr Thr Asp Ser Asp Glu Ile Ser His Ile Val
            40                  45                  50

aat cgt att gct cct cag gat gaa aaa cca aca aca aat tct atg ctg
606
Asn Arg Ile Ala Pro Gln Asp Glu Lys Pro Thr Thr Asn Ser Met Leu
        55                  60                  65

gac atg tgg att ggt gaa act gct att cct tgt act ccc agt gta gca
654
Asp Met Trp Ile Gly Glu Thr Ala Ile Pro Cys Thr Pro Ser Val Ala

70                          75                          80

aaa gga aaa tca aga gca aaa atc agc tgc aca aag tta aaa aca caa
702
Lys Gly Lys Ser Arg Ala Lys Ile Ser Cys Thr Lys Leu Lys Thr Gln
 85                          90                          95                         100

agt caa gaa gaa gaa ctt atg aaa ctg gct aaa caa ttt gat aaa aat
750
Ser Gln Glu Glu Glu Leu Met Lys Leu Ala Lys Gln Phe Asp Lys Asn
                        105                         110                         115

atg gaa gag cta gat gtg att caa gag caa aac aag agg aat tat gat
798
Met Glu Glu Leu Asp Val Ile Gln Glu Gln Asn Lys Arg Asn Tyr Asp
            120                 125                 130

ttt acc cag atg att tca gaa aca gag att tta agt aat tat aaa gat
846
Phe Thr Gln Met Ile Ser Glu Thr Glu Ile Leu Ser Asn Tyr Lys Asp
        135                 140                 145

aat ata cag atg tgg tca tta cat aat ata gtt ccc gaa ata gat aat
894
Asn Ile Gln Met Trp Ser Leu His Asn Ile Val Pro Glu Ile Asp Asn
    150                 155                 160

gct aca aaa aag cca atc aaa gaa aca cca aga tat ctg tgg caa ata
942
Ala Thr Lys Lys Pro Ile Lys Glu Thr Pro Arg Tyr Leu Trp Gln Ile
165                 170                 175                 180

atc aaa ata gca gtc aga agc cat ttg acc aaa ttg ctg aag cag cct
990
Ile Lys Ile Ala Val Arg Ser His Leu Thr Lys Leu Leu Lys Gln Pro
                185                 190                 195

tta atg cta ttt ttg atg gtc tac tca gaa atg tta gcg gac agt aag
1038
Leu Met Leu Phe Leu Met Val Tyr Ser Glu Met Leu Ala Asp Ser Lys
            200                 205                 210

cca aga act gcc aga ggc ttt tgg agc acc agt aat act acc ttt gta
1086
Pro Arg Thr Ala Arg Gly Phe Trp Ser Thr Ser Asn Thr Thr Phe Val
        215                 220                 225

aag aca aat gct ttg aaa gag gag aaa atc att act aat gaa act ctg
1134
Lys Thr Asn Ala Leu Lys Glu Glu Lys Ile Ile Thr Asn Glu Thr Leu
    230                 235                 240

gtc att gaa aaa ctg tca aat aaa acc cca cga tca ctt tct tct caa
1182
Val Ile Glu Lys Leu Ser Asn Lys Thr Pro Arg Ser Leu Ser Ser Gln
245                 250                 255                 260

gta gat aca ccc ata atg aca aaa tca tgt gtg act tcc tgt act aag
1230

Val Asp Thr Pro Ile Met Thr Lys Ser Cys Val Thr Ser Cys Thr Lys
                265                 270                 275

gag cca gaa act tct aat aag tac att gat gca ttt act aca agt gat
1278
Glu Pro Glu Thr Ser Asn Lys Tyr Ile Asp Ala Phe Thr Thr Ser Asp
            280                 285                 290

ttt gag gat gat tgg gaa aac tta cta ggt agt gaa cct ttt gct atg
1326
Phe Glu Asp Asp Trp Glu Asn Leu Leu Gly Ser Glu Pro Phe Ala Met
        295                 300                 305

caa aat atc gac atg cct gaa ctc ttt cct tct aaa aca gcc cat gtt
1374
Gln Asn Ile Asp Met Pro Glu Leu Phe Pro Ser Lys Thr Ala His Val
    310                 315                 320

act gat caa aag gaa att tgt acc ttt aat agt aaa act gtt aaa aat
1422
Thr Asp Gln Lys Glu Ile Cys Thr Phe Asn Ser Lys Thr Val Lys Asn
325                 330                 335                 340

acg tca aga gca aat aca agt cca gat gcc agg tta gga gat tca aaa
1470
Thr Ser Arg Ala Asn Thr Ser Pro Asp Ala Arg Leu Gly Asp Ser Lys
                345                 350                 355

gta tta caa gat ctt tct tca aag aca tat gac aga gaa tta ata gat
1518
Val Leu Gln Asp Leu Ser Ser Lys Thr Tyr Asp Arg Glu Leu Ile Asp
            360                 365                 370

gca gaa tat aga ttt tca cca aat tca aat aaa tca aac aaa tta tcc
1566
Ala Glu Tyr Arg Phe Ser Pro Asn Ser Asn Lys Ser Asn Lys Leu Ser
        375                 380                 385

act gga aat aaa atg aaa ttt gag aac tct tcc aat aaa att gtt att
1614
Thr Gly Asn Lys Met Lys Phe Glu Asn Ser Ser Asn Lys Ile Val Ile
    390                 395                 400

caa gac gaa att caa aat tgt ata gtt aca tct aat ctg aca aaa ata
1662
Gln Asp Glu Ile Gln Asn Cys Ile Val Thr Ser Asn Leu Thr Lys Ile
405                 410                 415                 420

aag gaa gat att ctt act aac tct act gaa gct tct gaa agg aag tca
1710
Lys Glu Asp Ile Leu Thr Asn Ser Thr Glu Ala Ser Glu Arg Lys Ser
                425                 430                 435

gct ttg aac aca agg tat tct aat gaa cag aaa aat aag tgc att tta
1758
Ala Leu Asn Thr Arg Tyr Ser Asn Glu Gln Lys Asn Lys Cys Ile Leu
            440                 445                 450

aat cag tct att aaa gcc cct gtt aat act gat ctt ttt ggc tct gca
1806
Asn Gln Ser Ile Lys Ala Pro Val Asn Thr Asp Leu Phe Gly Ser Ala
        455                 460                 465

aat cta ggc agt aaa acc agt gtt agt aac cca aat cag act agt gca
1854
Asn Leu Gly Ser Lys Thr Ser Val Ser Asn Pro Asn Gln Thr Ser Ala
    470                 475                 480

tca aaa gta ggt tct ttc ttt gat gat tgg aat gat ccc tca ttt gcc
1902
Ser Lys Val Gly Ser Phe Phe Asp Asp Trp Asn Asp Pro Ser Phe Ala
485                 490                 495                 500

aat gaa att att aaa gca tgt cat caa tta gat aat acc tgg gaa gca
1950
Asn Glu Ile Ile Lys Ala Cys His Gln Leu Asp Asn Thr Trp Glu Ala
                505                 510                 515

gat gat gta gat gat gat ttg ttg tac caa gca tgt gat gat att gaa
1998
Asp Asp Val Asp Asp Asp Leu Leu Tyr Gln Ala Cys Asp Asp Ile Glu
            520                 525                 530

aga cta act cag caa caa gac att aga aag gac agt aag aca tca gaa
2046
Arg Leu Thr Gln Gln Gln Asp Ile Arg Lys Asp Ser Lys Thr Ser Glu
        535                 540                 545

agt ata tgt gag atc aat aat aat tcc gaa cat gga gcc aaa cta act
2094
Ser Ile Cys Glu Ile Asn Asn Asn Ser Glu His Gly Ala Lys Leu Thr
    550                 555                 560

cag caa caa gac att aga aag gac agt aag aca tca gaa agt ata tgt
2142
Gln Gln Gln Asp Ile Arg Lys Asp Ser Lys Thr Ser Glu Ser Ile Cys

565                 570                 575                 580

gag atc aat aat aat tcc gaa cat gga gcc aaa aac atg ttt gct ata
2190
Glu Ile Asn Asn Asn Ser Glu His Gly Ala Lys Asn Met Phe Ala Ile
                585                 590                 595

tct aaa caa gga agt aat ttg gta caa tca aag cat ttg aat cca ggc
2238
Ser Lys Gln Gly Ser Asn Leu Val Gln Ser Lys His Leu Asn Pro Gly
            600                 605                 610

agc att tca gtg cag aca tct ttg aca aat agc tca caa ata gat aag
2286
Ser Ile Ser Val Gln Thr Ser Leu Thr Asn Ser Ser Gln Ile Asp Lys
        615                 620                 625

cca atg aag atg gag aaa ggg gaa atg tat gga aat tct cca aga ttt
2334
Pro Met Lys Met Glu Lys Gly Glu Met Tyr Gly Asn Ser Pro Arg Phe
    630                 635                 640

tta ggt gcc aca aat ttg act atg tat tct aag atc tca aac tgt cag
2382
Leu Gly Ala Thr Asn Leu Thr Met Tyr Ser Lys Ile Ser Asn Cys Gln
645                 650                 655                 660

ata aat aat ctg cat gtg tct tat act aac act gat gtt cca ata caa
2430
Ile Asn Asn Leu His Val Ser Tyr Thr Asn Thr Asp Val Pro Ile Gln
                665                 670                 675

gtg aat agt tcc aaa ttg gtt ctt cca gga agt tca agt ttg aat gta
2478
Val Asn Ser Ser Lys Leu Val Leu Pro Gly Ser Ser Ser Leu Asn Val
            680                 685                 690

act tca gat cat atg aat aca gaa att act act tat aag aag aaa ttg
2526
Thr Ser Asp His Met Asn Thr Glu Ile Thr Thr Tyr Lys Lys Lys Leu
        695                 700                 705

agt act aat cag cca tgc cat aag act gta aca gat gaa gct cag agc
2574
Ser Thr Asn Gln Pro Cys His Lys Thr Val Thr Asp Glu Ala Gln Ser
    710                 715                 720

aac ctt aac aca aca gtt gga ttt tca aag ttt aca ttt aca agg atg
2622
Asn Leu Asn Thr Thr Val Gly Phe Ser Lys Phe Thr Phe Thr Arg Met
725                 730                 735                 740

aaa aat tct cag att ctt tct cag ttt aat caa aat tgt ata act gga
2670
Lys Asn Ser Gln Ile Leu Ser Gln Phe Asn Gln Asn Cys Ile Thr Gly
                745                 750                 755

agt atg tct gat acc aaa att aca cag ggt gtg gag aaa aag aaa ggt
2718
Ser Met Ser Asp Thr Lys Ile Thr Gln Gly Val Glu Lys Lys Lys Gly
            760                 765                 770

gtc aac cca tta ctg gag gaa gct gtt gga cag caa tct ttg gtg aaa
2766
Val Asn Pro Leu Leu Glu Glu Ala Val Gly Gln Gln Ser Leu Val Lys
        775                 780                 785

ctt tct gaa tct ttg aaa caa tct tca aaa gag gaa gaa gag aaa aat
2814
Leu Ser Glu Ser Leu Lys Gln Ser Ser Lys Glu Glu Glu Glu Lys Asn
    790                 795                 800

aga aag tgt tct cct gaa gaa att cag aga aaa aga caa gaa gca ctg
2862
Arg Lys Cys Ser Pro Glu Glu Ile Gln Arg Lys Arg Gln Glu Ala Leu
805                 810                 815                 820

gtt cgg aga atg gct aaa gca cga gcc tca tct gta aat gca gct ccc
2910
Val Arg Arg Met Ala Lys Ala Arg Ala Ser Ser Val Asn Ala Ala Pro
                825                 830                 835

act tca ttt ctt taa tgaaatatta gttggaagac ttcacgaaga ctgctgataa
2965
Thr Ser Phe Leu
            840

ctatctgtga ttgataggaa attttttttc ttgatttctc tgtgagaaat gtaatgctga
3025

cttttataaa gcctggactt ctactttatt taataaatca atgtttgcaa tggtaaatga
3085

aacatttcct tggacatgta tttgaaagtc attaaataca aaagttttgg aaattcagga
3145

aagttagcaa ttatgtacgg atattataca gaggaaagta gttatatttt taaatgctat
3205

tattgcagag gatcatcaaa aaagaggtaa tctacgttat ttcctattct aatgtctttt
3265

cctaataaaa aacttcaact ttctaagtta gtagagactg tttttgtagt tttactttca
3325

ttaactgttg cttaagggtt tttatacact tccagatttt aattaaatac tttcatattt
3385

ctaggaacat aggttgatat gaaataaagg actttttatt tgtaaggaaa aaaaaaaaaa
3445

aaaaaactcg ag
3457

## Patentansprüche

1. Polypeptid mit einer Aminosäuresequenz gemäß SEQ. ID. Nr. 1.

2. Nukleinsäure mit einer Nukleotidsequenz gemäß SEQ. ID. Nr. 1 codierend ein Ewing Tumor spezifisches oder Ewing Tumor assoziierte Antigen.

3. Nukleinsäure codierend ein Polypeptid nach Anspruch 1.

4. Verwendung einer Nukleinsäure nach einem der Ansprüche 2 oder 3 zur Herstellung eines Oligonukleotids, eines Proteins oder Antikörpers zur Identifizierung und Diagnose von Ewing Tumorzellen.

5. Verwendung eines Polypeptids nach Anspruch 1 zur Herstellung eines Präparats für die Diagnose, Therapie oder Prävention vom Ewing Tumor.

6. Verwendung eines Polypeptids nach Anspruch 1 zur Herstellung eines Antikörpers gegen Ewing Tumor Zellen in vitro.

## Claims

1. Polypeptide having an amino acid sequence according to SEQ. ID. No. 1.

2. Nucleic acid having a nucleotide sequence according to SEQ. ID. No. 1, coding for a Ewing tumour-specific or Ewing tumour-associated antigen.

3. Nucleic acid, coding for a polypeptide according to Claim 1.

4. Use of a nucleic acid according to Claim 2 or 3 for producing an oligonucleotide, a protein or an antibody for identifying and diagnosing Ewing tumour cells.

5. Use of a polypeptide according to Claim 1 for producing a preparation for the diagnosis, therapy or prevention of a Ewing tumour.

6. Use of a polypeptide according to Claim 1 for producing an antibody against Ewing tumour cells in vitro.

## Revendications

1. Polypeptide comportant une séquence d'acides aminés conforme à l'ID SEQ. n° 1.

2. Acide nucléique comportant une séquence nucléotidique conforme à l'ID SEQ. n° 1 et qui code pour un antigène spécifique d'un sarcome d'Ewing ou associé à un sarcome d'Ewing.

3. Acide nucléique codant pour un polypeptide selon la revendication 1.

4. Utilisation d'un acide nucléique selon l'une des revendications 2 ou 3 pour préparer un oligonucléotide, une protéine ou un anticorps destiné(e) à l'identification et au diagnostic de cellules tumorales d'Ewing.

5. Utilisation d'un polypeptide selon la revendication 1 pour fabriquer une préparation destinée au diagnostic, à la thérapie ou à la prévention d'un sarcome d'Ewing.

6. Utilisation d'un polypeptide selon la revendication 1 pour fabriquer un anticorps dirigé contre des cellules tumorales d'Ewing in vitro.
